(19) Europäisches Patentamt
European Patent Office
Office européen des brevets

(11) Publication number: **0 135 429 B1**

(12) # EUROPEAN PATENT SPECIFICATION

(45) Date of publication of patent specification: **18.03.92**

(51) Int. Cl.[5]: **C07C 229/00**, C07C 227/00, C07K 7/04, A23L 1/236

(21) Application number: **84401665.9**

(22) Date of filing: **13.08.84**

(54) **The synthesis of cyclopropane amino acids and peptides.**

(30) Priority: **16.08.83 US 523808**
**03.08.84 US 636091**

(43) Date of publication of application:
**27.03.85 Bulletin 85/13**

(45) Publication of the grant of the patent:
**18.03.92 Bulletin 92/12**

(84) Designated Contracting States:
**AT BE CH DE FR GB IT LI LU NL SE**

(56) References cited:
**EP-A- 0 025 141**
**DE-A- 2 732 451**
**DE-A- 3 044 793**

**Monatshefte für Chemie, vol.103 , 1972, Springer-Verlag. I Bregovec et al. "Synthese der 1-Aminocyclopropan-1-carbonsäure" pages 288-291**

(73) Proprietor: **THE UNIVERSITY OF GEORGIA RESEARCH FOUNDATION, INC.**
**The University of Georgia, 612 Boyd**
**Graduate Studies Research Center**
**Athens, Georgia 30602(US)**

(72) Inventor: **Stammer, Charles H.**
**718 Riverhill Drive**
**Athens Georgia 30606(US)**

(74) Representative: **Eggert, Hans-Gunther, Dr.**
**Räderscheidtstrasse 1**
**W-5000 Köln 41(DE)**

EP 0 135 429 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The field of the invention is a new class of amino acid derivatives which in large measure have never been described in the literature and which were first synthesized into a peptide which exhibited unexpected stability against hydrolytic cleavage and degradation by organic acids and enzymes. A specific field of use for the new product is in a beverage composition which includes a novel peptide sweetener which is stable to acid hydrolysis by such organic acids as citric and fumaric acids and enzyme degradation and cleavage to give prolonged sweetness to the product. Food compositions are also described which employ the new peptides as ingredients.

The only process described in the literature and related to the novel process disclosed herein is the addition of compound (I) wherein $R_l = R_2$ = hydrogen to compound (2) to form a 1-aminocyclopropane-1-carboxylic acid or a cyclopropylamino acid (4) to wit:

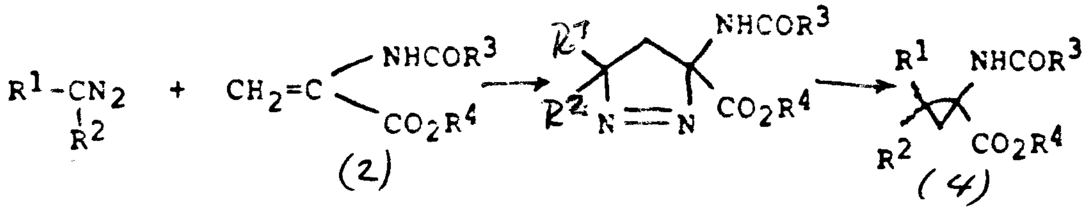

(2) (4)

wherein the $R^1$, $R^2$, $R^3$ and $R^4$ are as defined below, as reported by Bregovec and T. Jakovcic, Monatshefte für Chemie, 1972 103, 288. The commonly known addition of $CH_2N_2$ to unsaturated azlactones (M. Bernabe, et al, Ann de Quimica 1972, 68, 501, 1055; Eur. J. Med. Chem. 1979, 14, 33 (1979); and Synthesis Comm. 1977, 191; J. Hetrocyclic Chem. 1983, 20, 607; Pages, R.A. Burger, A; J. Medicinal Chemistry 1966, 9, 766; and 10, 435, (1967); Awad, W. I. et al Tetrahedron, 1964, 20, 891) is similar but not the same process and product of the present invention. The present synthesis requires the addition of a substituted diazomethane specifically to a dehydroalanine derivative which must be synthesized for this purpose to form a 5-substituted pyrazoline. Contrary to this the initial product formed in the process described in the prior art literature is a 4-substituted pyrazoline which is then converted into the 1-aminocyclopropane-1-carboxylic acid.

Other reported synthesis of 1-aminocyclopropane-1-carboxylic acid analogs of the present invention are reported by V. Schollkopf, R. Harms and D. Hoppe, Liebigs Ann. Chem. 611, (1973); also Martinez-Garcia, F.H. Cano and S. Garcia-Blanco, Acta Crystallographia, 31 (1980), Sect. A. Suppl. S103, also A. Ichihara, K. Shiraishi and S. Sakamura, Tetrahedron Letters 269, (1977) for synthesis of coronamic acid.

As to prior synthesis attempts at peptides containing 1-aminocyclopropane-1-carboxylic acid, see F.H.C. Stewart, Australian Jour. of Chemistry, 34 pp 2413 (1981).

As to foreign patents Spanish Patent No. 448,771 issued July 16, 1977 discloses a 1-aminocyclopropane-3-phenyl-1-carboxylic acid and 1-aminocyclopropane(parahydroxyphenyl)-1-carboxylic acid.

A cyclopropylphenylalanine has been described in the literature by King et al "Synthesis of Racemic (E) and (Z)-1-amino-2-phenylcyclopropanecarboxylic Acid: (E) and (Z) Cyclopropylphenylalanine" Jour. of organic Chem. 1982, 47, 3270-3273. In addition United States Patent 3,313,842 to Kaiser et al issued in 1967 discloses phenylcyclopropanecarboxylic acids and esters as hypotensive agents. U.S. Patent 3,050,559 to Burger discloses cyclopropyl amines. Also U.S. Patent 4,298,760 describes an improved process for preparation of 1-aminocyclopropane-1-carboxylic acid for use as a plant growth regulator. Kimura et al in Biochemical and Biophysical Research Communications, Vo. 115, p. 112-115 No.1 (1983) describes the synthesis of 1-aminocyclopropane-3-phenyl-1-carboxylic acid and its use in preparation of a stabilized peptide.

A cyclopropyl alanine derivative VAla and a dipeptide derivative comprising Tyr-VAla is also disclosed in DE-A-3 044 793 but this dipeptide was used for preparing N-adamantane-substituted tetrapeptide amides useful as analgesics.

DE-A-2 732 451 discloses peptides comprising an amino acid with a cycloalkyl group which can be used in pharmaceutical compositions.

As can be observed from the above prior art background several of the 1-aminocyclopropane-1-carboxylic acids are known materials and a peptide which employs one of these known amino acid analogues is reported in the prior art.

The process aspects of the present invention describe a method which generates chiral 1-aminocyclo-alkane-1-carboxylic acids directly. This means that if chirality is present in a dehydroalanine derivative optically active 1-aminocycloalkane-1-carboxylic acids can be prepared directly without the necessity of resolution. This is not possible in other reported prior art processes.

In the product aspects of the invention new stereo specific 1-aminocycloalkane-1-carboxylic acids are disclosed.

Another process aspect of the invention comprises a process for synthesizing an acid or enzyme stable peptide containing at least one stereo specific 1-aminocycloalkane-1-carboxylic acid residue. These totally unique peptides for the most part retain their initial biological activity but because of steric blocking or hinderance effected by the inclusion of the new claimed amino acid analogs as a substitute for one or more normal amino acid residues in the peptide chain the peptide becomes cleavage resistant.

As a result of their altered amino acid structure the peptide products of the invention do not degrade upon contact with hydrolytic enzymes or organic acids. Their enhanced stability is manifest by their new resistance to cleavage of the peptide linkages and methyl ester bonds. This stability has pharmacological advantages.

There are at least three products aspects to the invention. The first is the new and unique 1-aminocycloalkane-1-carboxylic acids described. These variant amino acids when substituted for normal amino acids in a peptide chain act to stabilize the peptide against cleavage by enzymes and hydrolysis by acids. The peptides so formed are themselves unique products having a distinctly different molecular structure and an added property of long term stability. A third product aspect of the invention resides in the preparation of a food product, for example, a beverage with long term sweetness when its peptide sweetener is replaced by the 1-aminocyclopropane-1-carboxylic acid modified peptide sweetener of the present invention.

Specific examples of each of these process and products aspects of the present invention will be set forth hereinbelow. It is of course clear that because of the magnitude of peptides known for a multitude of purposes in many end use areas of food chemistry, pharmacology, herbicide and pesticide end use applications, etc. the concrete embodiments to support all of the possible broad implications of the present invention are difficult to express in a single document. However, the basic concept and application of the same to a new food composition has been set forth herein to exemplify the broad notion of the invention.

It is therefore an object of the present invention to provide new stereo specific 1-aminocyclopropane-1-carboxylic acids It is a further object to provide new peptides containing at least one stereo specific 1-aminocyclopropane-1-carboxylic acid residue. It is a further object to provide an end product containing the peptide as an ingredient. These and other objects, aspects and advantages of this invention will become apparent from a consideration of the accompanying specification and claims:

## Best Mode for Carrying Out the Invention

The process embodiment of the present invention is carried out by allowing a diazo compound (1), in the presence or absence of a catalyst or light, to react with a dehydroalanine derivative (2). The initial reaction product may be a pyrazoline derivative (3) which is pyrolyzed, photolyzed or treated with a catalyst to give the 1-aminocyclopropane-1-carboxylic acid derivative `derivative (4) which is one of the product embodiments. The reaction is as shown in equations A and B, below:

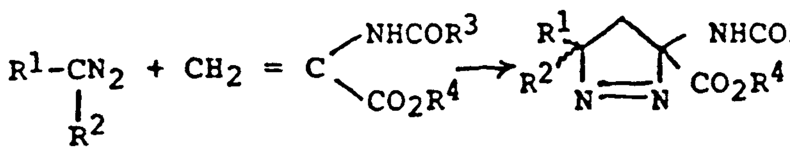
(Equation A);

(1) (2) (3)

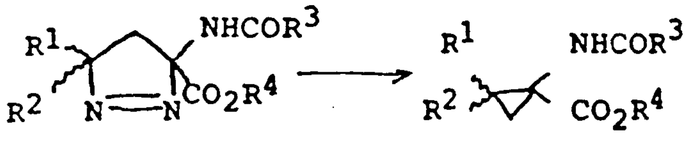
(Equation B);

(3) (4)

The product (4), a 1-aminocyclopropane-1-carboxylic acid derivative, derivative, may consist of a mixture of stereoisomers which are separable by physical means into the E- and Z diastereomers. Each of these

diastereomers consists of a pair (1R, 1S) of enantiomers which can be separated by standard resolution methods. Separation into E- and Z- diastereomers and separation of the enantiomers may occur either before or after deblocking the product (4).

In the diazo compound (1),

$R^1$ and $R^2$ are, in either order, a pair of substituents selected from the group consisting of $-CH_3$ and $-CH_3$, $-CH(CH_3)_2$ and -H, $-CO_2H$ and -H, $-CH_2CO_2H$ and -H, $-CH_2SCH_3$ and -H, $-(CH_2)_3NH_2$ and -H, $-CH_2CH_2NHC(=NH)NH_2$ and -H, $-(CH_2)_2NH_2$ and -H, $-C_6H_5$ and -H, -H and -H, 4-hydroxyphenyl and -H, 3,4-dihydroxyphenyl and -H, 3-indolyl and -H, 5-hydroxy-3-indolyl and -H, -OH and -H, -SH and -H, $-CH_2SH$ and -H, $-CH_2OH$ and -H, and 4(5)-imidazolyl and -H.

In dehydroalanine compound (2), $R^3$ can be any alkyl or aromatic group or alkoxy or aryloxy group. $R^4$ can be any alkyl or aryl group. Preferred lower alkyl groups are methyl, ethyl, propyl, isopropyl and butyl.

The solvent used in the reaction (A) can be any aprotic solvent, such as $CHCl_3$, $CH_2Cl_2$, tetrahydrofuran, dioxane, diethyl ether, etc. or protic solvent such as methanol or ethanol.

The reaction temperature (first step) is 0-30 °C and that of the second step may be 0°-150 °C. A solvent such as benzene or toluene or the like may be used in the second step.

For purposes of obtaining the free 1-aminocyclopropane-1-carboxylic acid (AA), compound (4) may be C-terminal deblocked, depending on the nature of $R_3$, by standard procedures such as saponification or hydrogenolysis giving the acid (5) as shown in equation C, below:

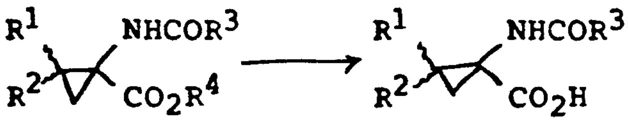

(4) (5)

(Equation C);

and N-terminal deblocking of (5) by the use of anhydrous acid, dry HCl or $CF_3CO_2H$, by hydrogenolysis or by hydrolysis, depending on the nature of $R^3$, can be accomplished by standard procedures as shown in equation D, below:

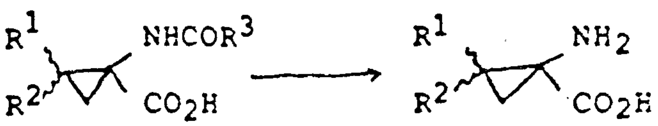

(5) (AA)

(Equation D)

Deblocking of the amino group (N-terminal deblocking) as shown in equation E, below, may precede deblocking of the carboxyl group (C-terminal deblocking) (equation C) resulting in a 1-aminocyclopropane-1-carboxylic acid.

A - PREFERRED EMBODIMENT - METHOD OF PREPARING 1-aminocyclopropane-1-carboxylic acid

One facet of this process invention thus discloses process for synthesizing a 1-aminocyclopropane-1-carboxylic acid selected from the group consisting of (1S)-E-, (1R)-E-, (1S)-Z-, (1R)-Z-, (1S)-, (1R)-, (1RS)-E-, and (1RS)-Z- isomers with respect to the carbon atom in 1-position wherein the 1-aminocyclopropane-1-carboxylic acid is selected from the group consisting of 1-aminocyclopropane-1-carboxylic acids analogs, derivatives, and cogeners thereof comprising the following steps:

(a) reacting a diazo compound having the formula $R^1R^2CN_2$

wherein $R^1$ and $R^2$ are, in either order, a pair of substituents selected from the group consisting of $-CH_3$ and $-CH_3$, $-CH(CH_3)_2$ and -H, $-CO_2H$ and -H, $-CH_2CO_2H$ and -H, $-CH_2SCH_3$ and -H, $-(CH_2)_3NH_2$ and -H, $-CH_2CH_2NHC(=NH)NH_2$ and -H, $-(CH_2)_2NH_2$ and -H, $-c_6H_5$ and -H, -H and -H, 4-hydroxyphenyl and -H, 3,4-dihydroxyphenyl and -H, 3-indolyl and -H, 5-hydroxy-3-indolyl and -H, -OH and -H, -SH and -H, $-CH_2SH$ and -H, $-CH_2OH$ and -H, and 4(5)-imidazolyl and -H. with a dehydroalanine derivative having the formula (3)

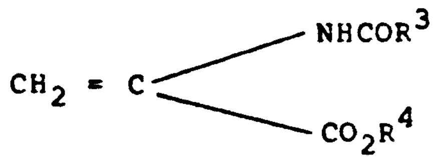

(3)

wherein $R^3$ is selected from the group consisting of an alkyl group, an aromatic group, an alkoxy group, and an aryloxy group and wherein $R^4$ is selected from the group consisting of an alkyl group and an aryl group to produce an initial reaction product;

(b) decomposing the initial reaction product (3) to produce a 1-aminocyclopropane-1-carboxylic acid derivative having the formula

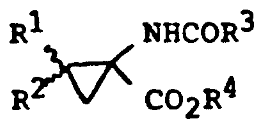

(4)

wherein the 1-aminocyclopropane-1-carboxylic acid derivative is a mixture of stereoisomers;

(c) separating the mixture of stereoisomers by physical means into E- and Z-diastereomers wherein the E- and Z-diastereomers comprise a pair of enantiomers;

(d) separating the pair of enantiomers by standard resolution means to produce a stereo specific 1-aminocyclopropane-1-carboxylic acid derivative;

(e) deblocking the initial reaction product to produce a stereo specific 1-aminocyclopropane-1-carboxylic acid having the formula

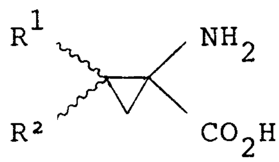

If $R^1$ or $R^2$ contain acidic groups selected from the group consisting of carboxyl, mercapto, and phenolic hydroxyl, $R^1$ and $R^2$ are blocked by standard means to protect such groups during the process. Synthesis of the following 1-aminocyclopropane-1-carboxylic acids requires such blocking; aspartic acid, tyrosine, 3-4-dihydroxyphenylalanine (DOPA), 5-hydroxytryptophan, cysteine, and homocysteine. If $R^3$ or $R^4$ of the dehydroalanine derivative is optically active, an optically stereo specific 1-aminocyclopropane-1-carboxylic acid can be produced without step (d) of the process. The diazo compound is reacted with the dehydroalanine derivative in the presence or absence of a catalyst and in the presence or absence of light. Pyrolisis, photolysis, or catalytic decomposition can be used to decompose the initial reaction product. A solvent selected from the group consisting of an aprotic solvent and a protic solvent can be used in the process. The step producing the initial reaction product is carried out at a temperature range of 0°C through 30°C, and the step producing the 1-aminocyclopropane-1-carboxylic acid derivative is carried out at a temperature range of 0°C through 150°C. When pyrolisis is used to decompose the initial reaction product, a solvent selected from the group consisting of benzene, toluene, and a similar solvent can be used in the step producing the 1-aminocyclopropane-1-carboxylic acid derivative. If the stereo specific 1-aminocyclopropane-1-carboxylic acid derivative is C-terminal deblocked to produce a stereo specific N-protected-1-aminocyclopropane-1-carboxylic acid having the formula

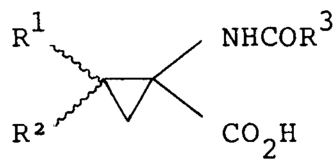

(5)

saponification or hydrogenolysis can be used to C-terminal deblock the stereo specific N-protected-1-aminocyclopropane-1-carboxylic acid derivative. After C-terminal deblocking, the stereo specific N-protected-1-aminocyclopropane-1-carboxylic acid is N-terminal deblocked to produce the stereo specific 1-aminocyclopropane-1-carboxylic acid. Anhydrous acid, the stereo specific N-protected-1-aminocyclopropane-1-carboxylic acid, dry hydrogen chloride, trifluoroacetic acid, hydrogenolysis, the stereo specific N-protected-1-aminocyclopropane-1-carboxylic acid, or hydrolysis can be used to N-terminal deblock the stereo specific N-protected-1-aminocyclopropane-1-carboxylic acid. N-terminal deblocking can precede carboxyl terminal deblocking to produce the stereo specific amino acid. Steps (c) and (d) or the process described above can be carried out before or after deblocking.

## PEPTIDE SYNTHESIS USING NEW AMINO ACIDS

For purposes of preparing peptides, compound (5), $R^3$ = $OCH_2Ph$ or $OC(CH_3)_3$, is prepared. Standard coupling methods, mixed anhydride, carbodiimide, etc., are used to couple (5) with C-terminal blocked amino acids or peptides. N-terminal deblocking of (4) to give (6) is accomplished using anhydrous acids, dry HCl or $CF_3CO_2H$, or hydrogenolysis depending on the natures of $R^3$ and $R^4$ as shown in equation E, below:

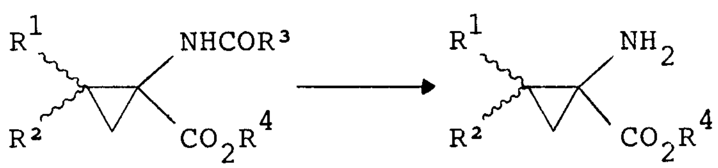

(Equation E)

(4) (6)

Compound (6) is useful in coupling with N-blocked carboxyl-activated amino acids or peptides to form desired peptides.

## PREFERRED EMOBODIMENTS - METHODS OF PEPTIDE SYNTHESIS

This invention discloses a process for synthesizing peptides having at least two amino acid residues selected from the group consisting of D- or L-isomers of amino acid residues wherein amino acid residues are selected from the group consisting of amino acid residues, analogs, derivative, and congeners thereof end wherein at least one amino acid residue is a stereo specific 1-aminocyclopropane-1-carboxylic acid residue selected from the group consisting of 1-aminocyclopropane-1-carboxylic acid residues, analogs, derivatives, and congeners thereof comprising the following steps:

(a) synthesizing the 1-aminocyclopropane-1-carboxylic acid derivative utilizing steps (a) and (b) of the process described above for synthesizing stereo specific 1-aminocyclopropane-1-carboxylic acid

(b) separating the stereo specific 1-aminocyclopropane-1-carboxylic acid utilizing steps (c) and (d) of the process described above for synthesizing stereo specific 1-aminocyclopropane-1-carboxylic acids

(c) deblocking the stereo specific 1-aminocyclopropane-1-carboxylic acid derivative by standard means to produce an N-terminal blocked stereo specific 1-aminocyclopropane-1-carboxylic acid

(d) coupling the N-terminal blocked stereospecific 1-aminocyclopropane-1-carboxylic acid with a C-terminal blocked amino acid or peptide; and

(e) repeating the above steps as necessary to produce a desired peptide. Step (c), the deblocking, can be carried out before step (b), the separation step.

An alternate process for synthesizing peptides utilizes stereo specific 1-aminocyclopropane-1-carboxylic

acids generated as described above comprises the following steps:

(a) N-terminal blocking the stereo specific 1-aminocyclopropane-1-carboxylic acid by standard means to produce the N-terminal blocked stereo specific 1-aminocyclopropane-1-carboxylic acid

(b) coupling the N-terminal blocked stereo specific 1-aminocyclopropane-1-carboxylic acid with a C-terminal blocked amino acid or peptide; and

(c) repeating the above steps as necessary to produce a desired peptide having at least two and not more than twenty amino acid residues selected from the group consisting of D- or L-isomers of amino acid residues wherein amino acid residues are selected from the group consisting of amino acid residues, analogs, derivatives, and congeners thereof and wherein at least one amino acid residue is a stereo specific 1-aminocyclopropane-1-carboxylic acid residue selected from the group consisting of 1-aminocyclopropane-1-carboxylic acid residues, analogs, derivatives, and congeners thereof.

A second alternate process for synthesizing peptides having at least two amino acid residues selected from the group consisting of D- or L-isomers of amino acid residues wherein amino acid residues are selected from the group consisting of amino acid residues, analogs, derivatives, and congeners thereof and wherein at least one amino acid residue is a stereo specific 1-aminocyclopropane-1-carboxylic acid residue selected from the group consisting of 1-aminocyclopropane-1-carboxylic acids residues, analogs, derivatives, and congeners thereof comprises the following steps:

(a) synthesizing a 1-aminocyclopropane-1-carboxylic acid utilizing steps (a), (b), and (e) of the process described above for synthesizing stereo specific 1-aminocyclopropane-1-carboxylic acid,

(b) N-terminal blocking the 1-aminocyclopropane-1-carboxylic acid by standard means to produce a N-terminal blocked 1-aminocyclopropane-1-carboxylic acid;

(c) separating the stereo specific 1-aminocyclopropane-1-carboxylic acid utilizing steps (c) and (d) of the process described above for synthesizing stereo specific 1-aminocyclopropane-1-carboxylic acids;

(d) coupling the N-terminal blocked stereospecific 1-aminocyclopropane-1-carboxylic acid with a C-terminal blocked amino acid or peptide; and

(e) repeating the above steps as necessary to produce a desired peptide.

Step (c) of this second alternate process for synthesizing peptides can be carried out before step (b) of same.

A third alternate process for synthesizing peptides having at least two amino acid residues selected from the group consisting of D- or L-isomers of amino acid residues wherein amino acid residues are selected from the group consisting of amino acid residues, analogs, derivatives, and congeners thereof and wherein at least one amino acid residue is a stereo specific 1-aminocyclopropane-1-carboxylic acid residue selected from the group consisting of 1-aminocyclopropane-1-carboxylic acid residues, analogs, derivatives, and congeners thereof comprises the following steps:

(a) synthesizing the 1-aminocyclopropane-1-carboxylic acid derivative utilizing steps (a) and (b) of the process described above for synthesizing stereo specific 1-aminocyclopropane-1-carboxylic acids,

(b) separating the stereo specific 1-aminocyclopropane-1-carboxylic acid utilizing steps (c) and (d) of the process described above for synthesizing stereo specific 1-aminocyclopropane-1-carboxylic acids;

(c) N-terminal deblocking the stereo specific 1-aminocyclopropane-1-carboxylic acid derivative by standard means to produce a C-terminal blocked amino acid;

(d) coupling the C-terminal blocked stereo specific 1-aminocyclopropane-1-carboxylic acid with a N-terminal blocked amino acid or peptide; and

(e) repeating the above steps as necessary to produce a desired peptide.

Step (c) of this third alternate process for synthesizing peptides can be carried out before step (b) of same.

A fourth alternate process for synthesizing peptides utilizing stereo specific 1-aminocyclopropane-1-carboxylic acids generated as described above and comprising the following steps:

(a) C-terminal blocking the stereo specific 1-aminocyclopropane-1-carboxylic acid by standard means to produce the C-terminal blocked stereo specific 1-aminocyclopropane-1-carboxylic acid,

(b) coupling the C-terminal blocked stereo specific 1-aminocyclopropane-1-carboxylic acid with a N-terminal blocked amino acid or peptide; and

(c) repeating the above steps as necessary to produce a desired peptide having at least two and not more than twenty amino acid residues selected from the group consisting of D- or L-isomers of amino acid residues wherein amino acid residues are selected from the group consisting of amino acid residues, analogs, derivatives, and congeners thereof and wherein at least one amino acid residue is a stereo specific 1-aminocyclopropane-1-carboxylic acid residue selected from the group consisting of 1-aminocyclopropane-1-carboxylic acid residues, analogs, derivatives, and congeners thereof.

A fifth alternate process for synthesizing a peptide having at least two and not more than twenty amino acid residues selected from the group consisting of D- or L-isomers of amino acid residues wherein amino

acid residues are selected from the group consisting of amino acid residues, analogs, derivatives, and congeners thereof and wherein at least one amino acid residue is a stereo specific 1-aminocyclopropane-1-carboxylic acid residue selected from the group consisting of 1-aminocyclopropane-1-carboxylic acid residues, analogs, derivatives, and congeners thereof comprises the following steps:

(a) synthesizing a 1-aminocyclopropane-1-carboxylic acids utilizing steps (a), (b), and (e) of the process described above for synthesizing stereo specific 1-aminocyclopropane-1-carboxylic acids

(b) C-terminal blocking the 1-aminocyclopropane-1-carboxylic acid by standard means to produce a C-terminal blocked 1-aminocyclopropane-1-carboxylic acid

(c) separating the C-terminal blocked stereo specific 1-aminocyclopropane-1-carboxylic acid utilizing steps (c) and (d) of the process described above for synthesizing stereo specific 1-aminocyclopropane-1-carboxylic acids

(d) coupling the C-terminal blocked stereo specific 1-aminocyclopropane-1-carboxylic acid with a N-terminal blocked amino acid or peptide; and

(e) repeating the above steps as necessary to produce a desired peptide.

Step (c) of this fifth alternate process for synthesizing peptides can be carried out before step (b) of same.

A sixth alternate process for synthesizing peptides utilizing stereo specific amino acids generated as described above, wherein $R^3$ or $R^4$ of the dehydroalanine derivative is optically active and step (d) of such process described above for synthesizing stereo specific 1-aminocyclopropane-1-carboxylic acids is not required, comprises the following steps:

(a) N-terminal blocking the stereo specific 1-aminocyclopropane-1-carboxylic acid by standard means to produce the N-terminal blocked stereo specific 1-aminocyclopropane-1-carboxylic acid

(b) coupling the N-terminal blocked stereo specific 1-aminocyclopropane-1-carboxylic acid with a C-terminal blocked amino acid or peptide; and

(c) repeating the above steps as necessary to produce a desired peptide having at least two and not more than twenty amino acid residues selected from the group consisting of D- or L-isomers of amino acid residues wherein amino acid residues are selected from the group consisting of amino acid residues, analogs, derivatives, and congeners thereof and wherein at least one amino acid residue is a stereo specific 1-aminocyclopropane-1-carboxylic acid residue selected from the group consisting of 1-aminocyclopropane-1-carboxylic acid residues, analogs, derivatives, and congeners thereof.

A seventh alternate process for synthesizing peptides utilizes stereo specific amino acids generated as described above, wherein $R^3$ or $R^4$ of the dehydroalanine derivative is optically active and step (d) of such process described above for synthesizing stereo specific 1-aminocyclopropane-1-carboxylic acids is not required, comprises the following steps:

(a) C-terminal blocking the stereo specific 1-aminocyclopropane-1-carboxylic acid by standard means to produce the C-terminal blocked stereo specific 1-aminocyclopropane-1-carboxylic acid

(b) coupling the C-terminal blocked stereo specific 1-aminocyclopropane-1-carboxylic acid with a N-terminal blocked amino acid or peptide; and

(c) repeating the above steps as necessary to produce a desired peptide having at least two and not more than twenty amino acid residues selected from the group consisting of D- or L-isomers of amino acid residues wherein amino acid residues are selected from the group consisting of amino acid residues, analogs, derivatives, and congeners thereof and wherein at least one amino acid residue is a stereo specific 1-aminocyclopropane-1-carboxylic acid residue selected from the group consisting of 1-aminocyclopropane-1-carboxylic acid residues, analogs, derivatives, and congeners thereof.

PREFERRED EMBODIMENTS - 1-aminocyclopropane-1-carboxylic acids

This invention discloses novel stereo specific 1-aminocyclopropane-1-carboxylic acid selected from the group consisting of (1S)-E-, (1R)-E-, (1S)-Z-, (1R)-Z-, (1S)-, (1R)-, (1RS)-E-, and (1RS)-Z-isomers with respect to the carbon atom in 1-position wherein the 1-aminocyclopropane-1-carboxylic acids are selected from the group consisting of cyclopropyl amino acids, analogs, derivatives, and congeners thereof having the formula

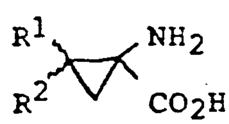

wherein $R^1$ and $R^2$ are, in either order, a pair of substituents selected from the group consisting of $-CH_3$

and $-CH_3$, $-CH(CH_3)_2$ and -H, $-CO_2H$ and -H, $-CH_2CO_2H$ and -H, $-CH_2SCH_3$ and -H, $-(CH_2)_3NH_2$ and -H, $-CH_2CH_2NHC(=NH)NH_2$ and -H, $-(CH_2)_2NH_2$ and -H, 3,4-dihydroxyphenyl and -H, 3-indolyl and -H, 5-hydroxy-3-indolyl and -H, -OH and -H, -SH and -H, $-CH_2SH$ and -H, $-CH_2OH$ and -H, and 4(5)-imidazolyl and -H. 1-aminocyclopropane-1-carboxylic acids (AA) which can be made according to this invention are shown in Table I, below.

**Table I**

1-aminocyclopropane-1-carboxylic acids

| $R^1$ | $R^2$ | VAA (novel compounds indicated by *) |
|---|---|---|
| $CH_3$ | $CH_3$ | *Valine |
| $(CH_3)_2CH$ | H | *Leucine |
| H | $(CH_3)_2CH$ | *Leucine |
| $CO_2H$ | H | *Aspartic acid |
| H | $CO_2H$ | *Aspartic acid |
| $CH_2CO_2H$ | H | *Glutamic acid |
| H | $CH_2CO_2H$ | *Glutamic acid |
| $CH_2CO$ | H | *pyroGlutamic acid |
| $CH_3SCH_2$ | H | *Methionine |
| H | $CH_3SCH_2$ | *Methionine |
| $NH_2(CH_2)_3$ | H | *Lysine |
| H | $NH_2(CH_2)_3$ | *Lysine |

| | | |
|---|---|---|
| $NH_2C(=NH)NHCH_2CH_2$ | H | *Arginine |
| H | $NH_2C(=NH)NHCH_2CH_2$ | *Arginine |
| $NH_2(CH_2)_2$ | H | *Ornithine |
| H | $NH_2(CH_2)_2$ | *Ornithine |
| $C_6H_5$ | H | Phenylalanine |
| H | H | Alanine |
| H | $C_6H_5$ | Phenylalanine |
| $4-HOC_6H_4$ | H | Tyrosine |
| H | $4-HOC_6H_4$ | Tyrosine |
| $3,4(HO)_2C_6H_3$ | H | *3,4 Dihydroxyphenylalanine |
| H | $3,4(HO)_2C_6H_3$ | *3,4 Dihydroxyphenylalanine |
| 3-indolyl | H | *Tryptophan |
| H | 3-indolyl | *Tryptophan |
| 5-hydroxy-3-indoyl | H | *Hydroxytryptophan |
| H | 5-hydroxy-3-indoyl | *Hydroxytryptophan |
| HO | H | *Serine |
| H | HO | *Serine |
| HS | H | *Cysteine |
| H | HS | *Cysteine |
| $HSCH_2$ | H | *Homocysteine |
| H | $HSCH_2$ | *Homocysteine |
| $HOCH_2$ | H | *Homoserine |
| H | $HOCH_2$ | *Homoserine |
| 4(5)-imidazolyl | H | *Histidine |
| H | 4(5)-imidazolyl | *Histidine |
| $(CH_2)_2$ | H | *Proline |
| $HO\ (CHCH_2)$ | H | *Hydroxyproline |

Except for valine, pyroGlutamic acid, proline, and proline analogs, the above 1-aminocyclopropane-1-carboxylic acids are shown as E- and Z-diastereomers. It is understood that all of the above listed 1-aminocyclopropane-1-carboxylic acids have (1R)- and (1S)-enantiomers.

PREFERRED EMBODIMENTS - POLYPEPTIDES

Peptides having at least two amino acids residues up to about 20 such residues with at least one of these being a 1-aminocyclopropane-1-carboxylic acid derivative are produced by the practice of one of the several methods of this invention as noted hereinabove. The amino acid residues which make up the peptide chain can be either D or L-isomers. The amino acid residue can be selected from the group consisting of (1S)-E, (1R)-E, (1S)-Z, (1R)-Z, (1S)- and (1R)-isomers of the stated amino acids in the peptide chain.

Selected examples of peptides capable of synthesis by means of the present invention and their specific utility is noted in the following Table II. The several stabilized peptides have a diverse field of use

ranging from the food additive dipeptide sweeteners of Group I to the complex bradykin or shock inhibitor peptides of Group VII or antihypertensives of Group V. The quantity of each peptide composition employed will vary with the specific intended use and mode of administration. In the case of the dipeptides VAsp-Phe •$OCH_3$ or Asp-V Phe •$OCH_3$ employed as a beverage sweetener a relatively large quantity will be required. These orally ingested peptide sweeteners have from 100 to 200 times the sweetening power of sucrose when substituted for the same. On the other hand relatively small amounts (50-100 mulligrams/kilo of body weight) of stabilized peptides of Group V to VIII principally employed in intravascular pharmaceutical administration are normally administered to achieve a therapeutic result.

In the following Table a brief compilation of selected new stabilized peptides ranging from $R^A$-$R^B$ ( a simple short chain sweetener bipeptide) to $R^A$-$R^I$ (a more complex central central nervous system regulatory hormone) is described. This list is only intended to illustrate the types of variety of polypeptides now capable of synthesis using the new and unique concepts described in the several specific preparations illustrated in the following Examples of a preferred mode of practice of the invention.

## Table II

| Group | Peptide Chain | Biological Function |
|---|---|---|
| I | $R^A-R^B$ | |
| | $\nabla$Asp-Phe•$OCH_3$ | Food and Sweetener |
| | Asp-$\nabla$Phe•$OCH_3$ | Beverage Sweetener |
| | $\nabla$Asp-$\nabla$Phe•$OCH_3$ | Food & Beverage Sweetener |
| | Asp-$\nabla$Ala•$OC_3H_7$ | Food & Beverage Sweetener |
| II | $R^A-R^B-R^C$ | |
| | $\nabla$Met-Leu-Phe | Bacteriostatic Agent |
| | Met-$\nabla$Leu-Phe | Bacteriostatic Agent |
| | pGln-His-$\nabla$Pro-$NH_2$ | Alstherapeutic |
| | pGln-$\nabla$His-Pro-$NH_2$ | Alstherapeutic |
| | $\nabla$pGln-His-Pro-$NH_2$ | Alstherapeutic |
| | Met-Leu-$\nabla$Phe | Bacteriostatic Agent |
| III | $R^A-R^B-R^C-R^D$ $R^E$ | Analgetics CNS Regulators |
| | Tyr-Gly-Gly-cbPhe-Leu | Analgetics CNS Regulators |
| | Tyr-Gly-Gly-cpPhe-Leu | Analgetics CNS Regulators |
| | Tyr-Gly-Gly-$\nabla$Phe-Leu | Analgetics CNS Regulators |
| | Tyr-Gly-Gly-$\nabla$Phe-Met | Analgetics CNS Regulators |
| | Tyr-D-Ala-Gly-$\nabla$Phe-Met | Analgetics CNS Regulators |
| | Tyr-D-Ala-Gly-$\nabla$Phe-Leu | Analgetics CNS Regulators |
| | $\nabla$Tyr-Gly-Gly-Phe-Leu | Analgetics CNS Regulators |
| | $\nabla$Tyr-D-Ala-Gly-Phe-Leu | Analgetics CNS Regulators |
| | $\nabla$Tyr-D-Ala-Gly-Phe-Met | Analgetics CNS Regulators |

| | |
|---|---|
| ∇Tyr-Gly-Gly-∇Phe-Leu | Analgetics CNS Regulators |
| ∇Tyr-Gly-Gly-∇Phe-Met | Analgetics CNS Regulators |
| Tyr-Gly-Gly-Phe-∇Leu | Analgetics CNS Regulators |
| Tyr-D-Ala-Gly-Phe-∇Leu | Analgetics CNS Regulators |
| ∇Tyr-D-ala-Gly-Phe-∇Leu | Analgetics CNS Regulators |
| Tyr-Gly-Gly-Phe-∇Met | Analgetics CNS Regulators |
| Tyr-D-Ala-Gly-Phe-∇Met | Analgetics CNS Regulators |
| ∇Tyr-D-Ala-Gly-Phe-∇Met | Analgetics CNS Regulators |
| ∇Tyr-Gly-Gly-Phe-∇Met | Analgetics CNS Regulators |
| ∇Tyr-Gly-Gly-Phe-∇Leu | Analgetics CNS Regulators |

**IV** $R^A-R^B-R^C-R^D-R^E-R^F$

| | |
|---|---|
| Ile-His-Pro-∇Phe-His-Leu | Blood Pressure Regulator |
| Ile-His-Pro-Phe-∇His-Leu | Blood Pressure Regulator |

**V** $R^A-R^B-R^C-R^D-R^E-R^F-R^G$

| | |
|---|---|
| Pro-Phe-His-∇Leu-Leu-Val-Tyr | Renin Inhibitors |
| Pro-Phe-His-Leu-∇Leu-Val-Tyr | Anti-hypertensives |
| Pro-∇Phe-His-Leu-∇Leu-Val-Tyr | |
| Pro-∇Phe-His-∇Leu-Leu-Val-Tyr | |

**VI** $R^A-R^B-R^C-R^D-R^E-R^F-R^G-R^H$

| | |
|---|---|
| Asp-Arg-Val-Tyr-Ile-His-Pro-∇Phe | Antihypertensive |
| Asp-Arg-Val-Tyr-Ile-∇His-Pro-Phe | Antihypertensive |
| Asp-Arg-Val-∇Tyr-Ile-His-Pro-Phe | Antihypertensive |
| Asp-Arg-∇Val-Tyr-Ile-His-Pro-Phe | Antihypertensive |
| Asp-∇Arg-Val-Tyr-Ile-His-Pro-Phe | Antihypertensive |
| ∇Asp-Arg-Val-Tyr-Ile-His-Pro-Phe | Antihypertensive |
| Asp-Arg-Val-∇Tyr-Ile-His-Pro-∇Phe | Antihypertensive |
| Asp-Arg-Val-∇Tyr-Ile-∇His-Pro-Phe | Antihypertensive |
| Asp-Arg-Val-Tyr-Ile-∇His-Pro-∇Phe | Antihypertensive |
| Sar-Arg-Val-∇Tyr-Ile-His-Pro-Phe | Antihypertensive |
| Sar-Arg-Val-Tyr-Ile-∇His-Pro-Phe | Antihypertensive |
| Sar-Arg-Val-Tyr-Ile-His-Pro-∇Phe | Antihypertensive |

| | |
|---|---|
| Sar-Arg-Val-Tyr-Ile-∇His-Pro-∇Phe | Antihypertensive |
| Sar-Arg-Val-∇Tyr-Ile-∇His-Pro-Phe | Antihypertensive |
| Sar-Arg-Val-∇Tyr-Ile-∇His-Pro-∇Phe | Antihypertensive |
| Sar-Arg-Val-∇Tyr-Ile-His-Pro-∇Phe | Antihypertensive |

| | | |
|---|---|---|
| VII | $R^A-R^B-R^C-R^D-R^E-R^F-R^G-R^H-R^I$ | |
| | Arg-Pro-Pro-Gly-Phe-Ser-Pro-∇Phe-Arg | Bradykin Inhibitors Shock Treatment |
| | Arg-Pro-Pro-Gly-∇Phe-Ser-Pro-Phe-Arg | Bradykin Inhibitors Shock Treatment |
| | Arg-Pro-Pro-Gly-∇Phe-Ser-Pro-∇Phe-Arg | Bradykin Inhibitors Shock Treatment |

In the above Table II the following list of abbreviations were employed:

| | |
|---|---|
| ∇ | = cyclopropane |
| cb | = cyclobutane |
| cp | = cyclopentane |
| ch | = cyclohexane |
| Phe·$OCH_3$ | = phenylalanine methyl ester |
| Arg | = arginine |
| Ala | = alanine |
| Asp | = aspartic acid |
| p Glu | = pyroglutamic acid |
| Gly | = Glycine |
| His | = Histidine |
| Ile | = isolencine |
| Leu | = leucine |
| Met | = methionine |
| Phe | = phenylalanine |
| Pro | = proline |
| Phe | = Phenylalanine |
| Pro | = proline |
| Pro $NH_2$ | = proline amide |
| Sar | = sarcosine |
| Ser | = serine |
| Tyr | = tyrosine |
| Val | = valine |

The following several specific Examples of preparations of the cyclopropane (V) amino acids as well as peptides where at one or more points in the chain they have replaced normal amino acids will serve to illustrate the invention claimed. Finally, the example of at least one improved food composition and one pharmaceutical composition will serve to illustrate the end use of the new modified amino acids and peptide compositions sought to be patented.

## EXAMPLE I

### Boc-Ser·OBzl($NO_2$) (1)

Boc-Ser:OH ().097 mol) was dissolved in ethyl acetate (140 ml) and p-nitrobenzyl bromide (21 g, 0.097 mol) followed by triethylamine (9.7 g, 0.097 mol) were added to the mixture, which was refluxed for 18 hr. After cooling, water (200 ml) was added to the reaction mixture, the layers were separated and the aqueous

layer was extracted with AcOEt. The extracts were washed with 5% $NaHCO_3$ soln. (100 ml), sat. NaCl soln, dried over $Na_2SO_4$ and evaporated in vacuo. The resulting pale yellow oil was dissolved in $Et_2O$ (100 ml) and kept in refrigerator for 4 hr. The colorless crystals were filtered by suction to give Boc-Ser·OBzl($NO_2$) (1) (18.0 g, 54.5%) as prisms; from the filtrate, a 2nd crop weighing 3.5 g was obtained (total yield 65%); mp 92-93 °C; IR: (KBr) 3320-3420 (NH,OH), 1745 (C=0), 1660 (C=0). NMR ($CDCl_3$) δ: 8.2 and 7.2 (AB d, J = 12 Hz, 4H, ArH). 5.5 (br, d, 1H, NH), 5.25 (s, 2H, $OCH_2$) 4.25-4.5 (m, 1H, CH), 3.90 (d, 2H, $CH_2OH$), 2.60 (br, 1H, OH), 1.38 (s, 9H, Boc).

EXAMPLE II

Boc-Dehydroalanine-p-nitrobenzyl ester (2)

a) Using-EDC

To a suspension of Boc-Ser·OBzl($NO_2$) (1) (6.0 g, 0.0176 mol) and CuCl (1.8 g, 0.018 mol) in $CHCl_3$ - (180 ml) was added 1-ethyl-3(3-dimethylamino-propyl)carbodiimide hydrochloride (EDC, 4.14 g, 0.0216 mol) at room temperature. The mixture was stirred overnight at room temperature during which time a brown oil separated. Water (150 ml) was added and the $CHCl_3$ layer was separated and washed with water, dried over $Na_2SO_4$, and evaporated in vacuo. The resulting solid was recrystallised from ethyl acetate-n-hexane to give colorless prisms 4.5 g (79.3%) of Boc-dehydroalanine-p-nitrobenzyl ester (2), mp 94-95 °C; IR; (KBr): 3420 (NH), 1710 (C=0), 1632 (C=0), 1600 (C=C). NMR ($CDCl_3$) δ: 8.19 and 7.5 (d, J = 12 Hz, 4H, ArH), 6.93 (br, s, 1H, NH), 6.20 (s, 1H, H-C=C), 5.75 (s, 1H, H-C=C), 5.30 (s, 2H, $OCH_2$).
Anal. Calcd. for $C_{15}H_{18}N_2O_6$ C: 55.0: H, 5.63: N: 8.69. Found: C: 55.85; H: 5.67; N: 8.65.

b) Using DCC

To a suspension of Boc-Ser·OBzl($NO_2$) (1) (6.0 g, 0.0176 mol) and CuCl (1.8 g, 0.018 mol) in $CHCl_3$ - (180 mol) was added dicyclohexylcarbodiimide (DCC: 4.9 g, 0.0216 mol) with ice cooling and the mixture was stirred for 3 days at room temperature. Water (200 ml) was added to the reaction mixture and additional stirring was continued for 3 min when the $CHCl_3$ layer was separated and the aqueous layer was extracted with $CHCl_3$. The combined $CHCl_3$ layer was washed with water, dried over $Na_2SO_4$ and evaporated in vacuo. The resulting residue was chromatographed by elution with benzene (silica gel 60-200 mesh, 40 g, Baker Analyzed) to give 4.1 g, (72.3%) of Boc-dehydroalanine-p-nitrobenzyl ester (2), mp 94-96 °C.

EXAMPLE III

p-Nitrobenzyl-3-t-butoxycarbonylaminopyrazoline-3-carboxylate (3)

To a solution of Boc-dehydroalanine-p-nitrobenzyl ester (2) (700 mg, 2.2 mmol) in $CH_2Cl_2$ (10 ml) was added dropwise ethereal $CH_2N_2$ solution (prepared from Diazald, 4.12 g, 19 mmol) in $Et_2O$ (40 ml) over a period of 40 min with ice cooling. After stirring for 1 hr at 0 5 °C, excess of $CH_2N_2$ was decomposed by addition of $CaCl_2$ at room temperature and the mixture was filtered. The filtrate was evaporated in vacuo to give a white solid and the solid was triturated with n-hexane and then filtered by suction to give 3 (710 mg: 88.5%), mp 79-80 °C. Recrystallizatiion from AcOEt-n-hexane gave pure p-Nitrobenzyl-3-t-butoxycarbonylaminopyrazoline-3-carboxylate (3) as colorless prisms, mp 84 °C (dec.); IR: (KBr) 3270 (NH), 1700-1740 (C=0), 1600 (N=H). NMR($CDCl_3$)δ: 8.2 and 7.45 (dd, 4H, ArH H), 6.38 (s, 1H, NH), 5.29 (s, 2H, $OCH_2$), 4.4-52 (m, 2H, $NCH_2$), 1.95-2.25 (m, 2H, $CH_2$), 1.40 (s, 9H, Boc).

Anal. Calcd. for $C_{16}H_{20}N_4O_6$: C: 52.74; H: 5.53; N: 15.38. Found: C: 52.62; H: 5.59; N: 15.34.

EXAMPLE IV

Boc-1-aminocyclopropane-1-carboxylic acid p-nitrobenzyl Ester (4)

A mixture of the pyrazoline, p-nitrobenzyl-3-t-butoxycarbonylamino-pyrazoline-3-carboxylate (3) (550 mg), and benzene (l0 ml) was refluxed for l.5 hr (bath temp., ca 90 °C) and the benzene was removed in vacuo to give a solid, which was recystallized from AcOEt-n-hexane to afford 508 mg of Boc-1-aminocyclopropane-1-carboxylic acid p-nitrobenzyl Ester (4) (l00%); mp ll7-ll8 °C; IR (KBr) 3350 (NH), l730

(C=0), I680 (C=0). NMR ($CDCl_3$) δ: 8.2 and 7.48 (AB d, 4H, ArH), 5.2 (s, 2H, $OCH_2$), 5.I5 (br, s, IH, NH), I.00-I.75 (m, 4H, $CH_2$x2), I.44 (s, 9H, Boc).

Anal. Calcd. for $C_{16}H_{20}N_2O_6$: C: 57.I4; H: 5.99; N: 8.33. Found: C: 57.05; H: 5.99; N: 8.3I.

EXAMPLE V

Boc-1-aminocyclopropane-1-carboxylic acid (5)

To a solution of Boc-1-aminocyclopropane-1-carboxylic acid p-nitrobenzyl Ester (4) (450 mg, I.34 mmol) in MeOH (20 ml) was added IN NaOH (2.6 ml, 2.6 mmol) at room temperature. After the mixture was stirred for 3 hr, water (I0 ml) was added and the MeOH was removed in vacuo. The aqueous residue was washed with $Et_2O$ to remove p-nitrobenzyl alcohol and the aqueous layer was separated, cooled in an ice bath and I0% citric acid solution was added to pH 3. The mixture was saturated with NaCl and extracted with AcOEt and the extracts were washed with sat. NaCl solution, dried over $Na_2SO_4$ and evaporated in vacuo to give a white solid which was recrystallised from AcOEt-n-hexane to afford 260 mg of Boc-1-aminocyclopropane-1-carboxylic acid (5) (96.3%) as colorless needles, mp 176-177°C (dec.); IR (KBr): 3230(NH), 1630-1680 (C=0). NMR ($CDCl_3$+DMSO) δ: 9.45 (br, s, 1H, COOH), 5.88 (br, s, 1H, NH) 1.3-1.7 (m, 2H, $CH_2$), 1.0-1.2 (m, 2H, $CH_2$), 1.50 (s, 9H, Boc).

Anal. Calcd. for $C_9H_{15}NO_4$ C: 53.72; H: 7.51; N: 6.96. Found: C: 53.59; H: 7.58; N: 6.88.

EXAMPLE VI

Diazoisobutane (6)

To a solution of isobutylurea (2.0 g, 0.017 mol) in HOAc/$H_2O$ (6:1) (6 ml) was added dropwise 4.8 M $NaNO_2$ solution (6 ml) with ice cooling over 1 hr. After stirring was continued for an additional 1 hr, water (20 ml) was added to the reaction mixture and the yellow crystals were extracted into $CHCl_3$. The extract was washed with water and evaporated at 25°C to dryness giving a yellow solid. The resulting crude nitroso compound was dissolved in $Et_2O$ (20 ml) and the solution was added dropwise to the mixture of 40% KOH solution (5.4 ml) and $Et_2O$ (20 ml) at -15°C to -20°C over a 1 hr period. The reaction mixture was stirred for 1 hr at the same temperature and the $Et_2O$ layer containing diazoisobutane (6) was separated and used in the next reaction immediately.

EXAMPLE VII

p-Nitrobenzyl 3-t-Butoxycarbonylamino-5-ispropylpyrazoline-3-carboxylate (7)

The ethereal diazoisobutane (6) was gradually added to a solution of Boc-dehydroalanine-p-nitrobenzyl ester (2) (967 mg, 3 mmol) in $CH_2Cl_2$ (15 ml) at -10°C to -15°C with stirring. After stirring for 1 hr at the same temperature, the solvent was evaporated in vacuo and the resulting residue was triturated with hexane and filtered by suction to give I.2 g of p-nitrobenzyl p-nitrobenzyl 3-t-butoxycarbonylamino-5-isopropylpyrazoline-3-carboxylate (7) (98.4%) mp 78-79°C (dec.). Recrystallization from AcOEt-n hexane gave colorless prisms having mp 87-89°C (dec.); IR (KBr) 3390(NH), I745(C=0), I690(C=0), I605 (N=N)-.NMR ($CDCl_3$) δ: 8.2 and 7.5 (d, d, 4H, ArH), 6.2 (br s, IH, NH); 5.30 (s, 2H, $OCH_2$), 4.8-5.2 (m, IH, CH-N=N), I.5-2.3 (m, 3H, $(CH_3)_2CH$ and $CH_2$), I.35 (s, 9H, Boc), I.00-I.30 (m, 3H, $CH_3$), 0.8-I.I0 (m, 3H, $CH_3$).

Anal. Calcd for $C_{19}H_{26}N_4O_6$: C: 56.I5; H: 6.45 N: I3.79. Found: C: 55.93; H: 6.53; N: I3.75.

EXAMPLE VIII

Boc-2-isopropyl-1-aminocyclopropane-1-carboxylic Acid p-Nitrobenzyl Ester (8)

The pyrazoline, p-nitrobenzyl 3-t-butoxycarbonylamino-5-isopropylpyrazoline-3-carboxylate (7) (I.I g, 2.7 mmol), was dissolved in benzene (20 ml), the solution was refluxed for 2 hr and evaporated in vacuo to give a white solid which was recrystallized form AcOEt-n-hexane giving colorless prisms of Boc-2-isopropyl-1-aminocyclopropane-1-carboxylic Acid p-Nitrobenzyl Ester (8) , 950 mg (95%), mp I39-I43°C, IR (KBr) 3360-(NH), I725(C=0), 1680 (C=O). NMR ($CDCl_3$ δ: 8.20 and 7.52 (d,d, 4H, ArH), 5.25 (s, 2H, $OCH_2$), 5.22 (br, s, IH,NH), I.2-I.8 (m, 4H, $CH_2$, CHx2), I.4 (s, 9H, Boc), 0.8-I.I5 (m, 6H, $(CH_3)_2CH$).

Anal. Calcd. for $C_{19}H_{26}N_2O_6$: C: 60.30; H: 6.93; N: 7.40. Found: C: 59.73; H, 7.05; N, 8.27.

EXAMPLE IX

Boc-2-isopropyl-1-aminocyclopropane-1-carboxylic Acid (9)

To a suspension of Boc-2-isopropyl-1-aminocyclopropane-1-carboxylic Acid p-Nitrobenzyl Ester (8) (300 mg, 7.9 mmol) in MeOH (20 ml) was added 2N NaOH soln. (7 ml) under ice cooling and the mixture was stirred for 3 hr at room temperature, the starting material gradually dissolved and the mixture turned yellow. Water (l0 ml) was added and the MeOH was evaporated in vacuo. The aqueous residue was washed with AcOEt, cooled in an ice bath and acidified by the addition of l0% citric acid to pH 3. The resulting white precipitate was extracted with AcOEt (3x20 ml) and the extract was washed sat. NaCl soln, dried over $Na_2SO_4$, and evaporated in vacuo.The resulting of Boc-2-isopropyl-1-aminocyclopropane-1-carboxylic Acid (9) was recrystallized from AcOEt-n-hexane to give l40 mg, (72.5%) as colorless prisms; mp l96-7°C (dec.); IR: (KBr) 3230(NH), l690(C=0), l645(C=0). NMR ($CDCl_3$ + DMSO-$d_6$) δ: 5.78 (s, lH, NH), l.2-l.8 (m, 4H, $CH_2$, CHx2), l.4 (s, 9H, Boc), 0.9-l.l (m, 6H, $(CH_3)_2CH$).

Anal. Calcd for $C_{l2}H_{2l}NO_4$: C: 59.24; H: 8.70; N: 5.76. Found: C: 59.28; H: 8.74; N: 5.72.

EXAMPLE X

Benzaldehyde p-Toluenesulfonyl Hydrazone (l0)

A mixture of benzaldehyde (5.25 g, 0.05 mol), p-toluenesulfonyl hydrazide (9.3 g, 0.05 mol) an dAcOH (20 ml) was stirred for l5 min at 70°C, and allowed to stand overnight at room temperature. $Et_2O$ (20 ml) was added to the mixture, the precipitated solid was tritrated with ether and the crystals were filtered by suction and washed with $Et_2O$ to give Benzaldehyde p-Toluenesulfonyl Hydrazone (l0), 9.7g (70.7%), mp l26-l28°C (dec.).

EXAMPLE XI

Boc-E-2-phenyl-1-aminocyclopropane-1-carboxylic acid p-Nitrobenzyl Ester (11)

Sodium (l38 mg, 6 mmol) was dissolved in ethylene glycol (l0 ml) and the tosylhydrazone, benzaldehyde p-toluenesulfonyl hydrazone (l0), 923 mg, 3 mmol) was added to the solution. When dissolution was completed, hexane (20 ml) was added and the mixture was refluxed for 20 min (bath temp. 85-90°C) with vigorous stirring. Then the mixture was cooled in an ice bath and the resulting red colored product was extracted with cold n-hexane (20 ml x 3). The combined extracts were washed with lN NaOH soln. (20 ml), sat. NaCl soln. (20 ml) and then dried over $Na_2SO_4$. After filtering, the pink filtrate was added to a mixture of Boc-dehydroalanine-p-nitrobenzyl ester (2) (322 mg, l mmol) in $CH_2Cl_2$ (l0 ml) over a period of l5 min at 0°C. The mixture was stirred overnight at room temperature and the red color disappeared. The solvent was evaporated in vacuo and the residue was triturated with n-hexane-ether. The resulting solid was filtered by suction to give Boc-E-2-phenyl-1-aminocyclopropene-1-carboxylic acid P-Nitrobenzyl Ester (11) (380 mg, 92.2%); mp ll5-6°C; IR: (KBr) 3390(NH), l7l5(C=O). NMR ($CDCl_3$) δ: 8.1 and 7.l (dd, 4H, ArH), 7.l-7.50 (m, 5H, ArH), 5.45 (br. s, lH, NH), 4.9 (s, 2H, $CH_2O$), 2.8-3.l (m, lH, CH), 2.0-2.4, l.2 l.8 (m, 2H, $CH_2$), l.45 (s, 9H, Boc).

EXAMPLE XII

Boc-E-2-phenyl-1-aminocyclopropane-1-carboxylic acid (12)

A mixture of Boc-E-2-phenyl-1-aminocyclopropane-1-carboxylic acid p-Nitrobenzyl ester (11) nitrobenzyl Ester (ll) (200 mg, 0.485 mmol), MeOH (l0 ml) and 2N NaOH soln. (3 ml) was stirred overnight at room temperature. Water (l0 ml) was added and the MeOH was evaporated in vacuo. The residue was washed with AcOEt and the aqueous layer was cooled in an ice bath and acidified with l0% citric acid to pH 3. The mixture was saturated with NaCl and extracted with AcOEt. The extract was washed with sat. NaCl soln, dried over $Na_2SO_4$ and evaporated in vacuo. The resulting solid was recrystallized from AcOEt-n-hexane to give Boc-E-2-phenyl-1-aminocyclopropane-1-carboxylic acid (12) (90 mg, 67.2%) as colorless prisms, mp l58-l60°C (dec.); NMR ($CDCl_3$) δ: 7.2-7.4 (m, 5H, ArH), 2.7-2.9 (m, lH, CH), 2.0-2.3 and l.5-l.7 (m, 2H, $CH_2$), l.5 (s, 9H, BOC). NMR identical with that of earlier sample.

EXAMPLE XIII

p-Nitrobenzyl 3-t-Butoxycarbonylamino-5-(N-tosylindol-3-yl)-pyrazoline-3-carboxylate (I3)

A solution of N-tosylindol-3-yl diazomethane (3 mml) in $CH_2Cl_2$ (20 ml) is added to a solution of Boc-dehydroalanine-p-nitrobenzyl ester (2) in 20 ml $CH_2Cl_2$ at -I5°C. After stirring I hr at -I5°C and 4 hr at 25°C, the solution is evaporated to dryness and the residue is triturated with hexane. The solid product, p-nitrobenzyl 3-t-butoxycarbonylamino-5-(N-tosylindol-3-yl)-pyrazoline-3-carboxylate (I3), is recrystallized from ethyl acetate-hexane to constant melting point. NMR ($DCDI_3$) δ: 4.8-5.4 (m, IH, CH-N=N).

EXAMPLE XIV

Boc-2(3-indolyl)-1-aminocyclopropane-1-carboxylic Acid p-nitrobenzyl Ester (14)

The pyrazoline, p-nitrobenzyl 3-t-butoxycarbonylamino-5-(N-tosylindol-3-yl)-pyrazolline-3-carboxylate (I3), (I mmole) is suspended in toluene (50 ml) and the mixture is refluxed until its NMR spectrum shows the presence of cyclopropane protons (δ3.0-3.5 and 0.8-I.2 ppm) and the loss of the pyrazoline peak at δ 5.0. (2 hr). The solution is evaporated and the residue of Boc-2(3-indolyl)-1-aminocyclopropane-1-carboxylic Acid p-nitrobenzyl Ester (14) is crystallized from ethylacetate-hexane. NMR ($DCDI_3$) δ: 2.7-3.0 (m, IH, CH), 2.0-2.3 and I.5-I.7 (m, 2H, $CH_2$).

EXAMPLE XV

p-Nitrobenzyl 3-t-Butoxycarbonyl-5-(3-chloropropyl)pyrazoline-3-carboxylate (I5)

4-Chlorobutyraldehyde tosylhydrazone (3 mmole) is added to a solution of sodium (6 mmole) in ethylene glycol (I5 ml). Hexane (25 ml) is added and the mixture is stirred 30 min at 90°C. After cooling, the diazo-compound is extracted into cold hexane (3 x 20) ml). The combined extracts are washed with IN NaOH (20 ml) and saturated NaCl solution (25 ml) and dried over anhyd. $Na_2SO_4$. After filtration, the filtrate is added to a mixture of Boc-dehydroalanine-p-nitroenzyl ester (2) (I mmole) in $CH_2Cl_2$ (I0 ml) at 0°C over a I5-minute period. After stirring at 25°C for I6 hr, the solution is evaporated and the residue is triturated with $Et_2O$-hexane. The solid pyrazoline, p-nitrobenzyl 3-t-butoxycarbonyl-5-(3-chloropropyl)-pyrazoline-3-carboxylate (I5), is crystallized from ethyl acetatehexane. NMR ($CDCl_3$) 4.5-5.4 (m, IH, CH-N=N).

EXAMPLE XVI

Boc-2-(3-chloropropyl)-1-aminocyclopropane-1-carboxylic Acid p-Nitrobenzyl Ester (16)

The pyrazoline, p-nitrobenzyl 3-t-butoxycarbonyl-5-(3-chloropropyl)-pyrazoline-3-carboxylate (I5), (I mmole) is suspended in toluene (50 ml) and the mixture is refluxed until its NMR spectrum shows the presence of cyclopropane protons ( δ3.0-3.5 and 0.8-I.2 pmm) and the loss of the pyrazoline peak at δ5.0 (2 hr). The solution is evaporated and the residue of Boc-2-(3-chloropropyl)-1-aminocyclopropane-1-carboxylic Acid p-Nitrobenzyl Ester (16) is crystallized from ethyl acetate-hexane. NMR ($CDCl_3$) δ:2.7-3.0 (m, IH, CH), 2.0-2.3 and I.5-I.7 (m, 2H, $CH_2$).

EXAMPLE XVII

Boc-2-(3-aminopropyl)-1-aminocyclopropane-1-carboxylic Acid p-Nitrobenzyl Ester (17)

The cyclopropyl alanine derivative Boc-2-(3-chloropropyl)-1-aminocyclopropane-1-carboxylic Acid p-Nitrobenzyl Ester (16) is treated with a IM solution of ammonia in isopropanol at 50°C in a sealed pressure bottle for 48 hrs. Evaporation of the solution gives a solid residue which is dissolved in warm ethyl acetate (5 ml) and the solution is washed with saturated NaCl solution (3x25 ml) and dried over anhyd. $Na_2SO_4$. Evaporation of the solution gives Boc-2-(3-aminopropyl)-1-aminocyclopropane-I-carboxylic Acid p-Nitrobenzyl Ester (17) which recrystallized from ethyl acetatehexane. NMR ($CDCl_3$) δ: I.2-I.8 (m, 5, $CH_2CH_2CH$), 0.8-I.I (m, 2H, $CH_2$), 2.5 (m, 2H, $CH_2NH_2$).

Example XVIII

p-Nitrobenzyl 3-t-Butoxycarbonylamino-5-(4-[2,4-dinitrophenoxyl]-phenyl)-pyrazoline-3-carboxylate (l8).

A solution of 4-(2,4-dinitrophenoxy)-phenyldiazomethane in $CH_2Cl_2$ (20 ml) is added to a solution of Boc-dehydroalanine-p-nitrobenzyl ester (2) in 20 ml $CH_2Cl_2$ at -l5 °C. After stirring l hr at -l5 °C and 4 hr at 25°, the solution is evaporated to dryness and the residue is triturated with hexane. The solid product, p-nitrobenzyl 3-t-butoxycarbonylamino-5-(4-[2,4-dinitro-phenoxyl]-phenyl)-pyrazoline-3-carboxylate (l8), is recrystallized from ethyl acetate-hexane to constant melting point. NMR ($CDCl_3$) δ: 4.8-5.4 (m, lH, CH-N=N).

Example XIX

Boc-0-2,4-dinitrophenyl-2-(4-hydroxyphenyl)-1-aminocyclopropane-1-carboxylic Acid p-Nitrobenzyl Ester (19)

The pyrazoline, p-nitrobenzyl 3-t-butoxycarbonylamino-5-(4-[2,4-dinitrophenoxyl]-phenyl)-pyrazoline-3-carboxylate (18), is suspended in toluene (50 ml) and the mixture is refluxed until its NMR spectrum shows the presence of cyclopropane protons ( δ3.0-3.5 and 0.8-1.2 ppm) and the loss of the pyrazoline peat at δ5.0 (2 hr). The solution is evaporated and the residue of Boc-0-2,4-dinitrophenyl-2-(4-hydroxyphenyl)-1-aminocyclopropane-1-carboxylic Acid p-Nitrobenzyl Ester (19) is crystallized from ethylacetate-hexane. NMR ($CDCL_3$ δ: 2.7-3.0 (m, lH, CH), 2.0-2.3 and 1.5-1.7 (m, 2H, $CH_2$).

Example XX

Boc-2-(4-hydroxyphenyl)-1-aminocyclopropane-1-carboxylic Acid p-Nitrobenzyl Ester (20)

A solution of Boc 0-2,4-dinitrophenyl cyclopropyl tyrosine p-nitrobenzyl ester (19) (l mmole) in DMF (2 ml) is treated with 2-mercaptoethanol (22 mmole). After l hr. at 25 °C the DMF was evaporated and the residue of Boc-2-(4-hydroxyphenyl)-1-aminocyclopropane-1-carboxylic Acid p-Nitrobenzyl Ester (20) is crystallized from ethyl acetate-hexane. NMR ($CDCl_3$) δ : 2.7-2.9 (m, lH, CH), 2.0-2.3, l.5-l.7 (m, 2H, $CH_2$).

Example XXI

Cbz-(1RS)- $\nabla^E$Phe-Leu·Ome, (2l).

A solution of isobutyl chloroformate (546 mg, 4 mmol) in chloroform (10 ml) was added dropwise to a solution of Boc-Ser·OBzl($NO_2$) (l) (1.24 g, 4 mmol) and N-methylmorpholine (404 mg, 4 mmol) in chloroform (30 ml) at 0.5 °C. After stirring for 20 min, a solution of Leu·OMe·HCl (1.45 g, 8 mmol) and N-methylmorpholine (0.81 g, 8 mmol) in chloroform (20 ml) was added at 10 °C. After stirring for 2 hr at 0.5 °C and then overnight at room temperature, the reaction mixture was washed with $H_2O$, 5% citric acid and 5% $NaHCO_3$ successively, and dried over $Na_2SO_4$. The solvent was evaporated in vacuo and the residue was recrystallized from ethylacetate-hexane to give CGz-(2RS)- $\nabla^E$ Phe-Leu·OMe, '21) (l.l3 g, 64.4%) as colorless needles, mp 94-97 °C. $R_f$(I) = 0.85, $R_f$(III) = 0.12.

Anal. Calcd. for $C_{25}H_{30}N_2O_5$: C, 68.47; H, 6.90; N, 6.39. Found: C, 68.50; H, 6.99; N, 6.38.

Separation of Cbz(1RS)- $\nabla^E$ Phe-Leu·OMe, (21) into Diastereomers by HPLC.

Cbz(1RS)- $\nabla^E$ Phe-Leu·OMe, (21) was separated into its diastereoisomers using HPLX ($C_{18}$ Lichrosorb, 20 cm x 0.46 cm, $CH_3CN$-$H_2O$ (55:45), 2 ml/min). The (1S) $\nabla^E$Phe isomer showed $t_R$=6.2 min and the (1R) $\nabla^E$Phe isomer showed $t_R$=8.1 min. This compound has utility in synthesis as an intermediate to form an enkephalin peptide which inhibits muscle contraction.

Example XXII

(1S)-$\nabla^e$Phe-Leu •OMe •TFA (22)

A solution of Cbz-(1S)-$\nabla^E$Phe-Leu •OMe (1.31 g, 3 mmol) and thioanisole (2 ml) in trifluoroacetic acid (TFA) (20 ml) was stirred at 0° for 3 hr, and then room temperature overnight. The TFA was removed under reduced pressure, and the residue was triturated with ether (30 ml) and the precipitated crystals were collected by suction and washed with ether to give (1S)-$\nabla^E$Phe-Leu •OMe •TFA (22) (1.14 g, 91.2%), mp

251-2° (dec.); $[\alpha]^D$-89.6° (C=0.5 $H_2O$). NMR ($CF_3CO_2H$-$CDCl_3$ (1:) δ : 0.67 (6H, br s ($CH_3$)), 0.78-1.40 (3H, m, $CH_2CH$), 2.28 (2H, d, J = 10 Hz, ), 3.50 (1H, t, J = Hz, $_H$-Δ), 3.85 (3H, s, $CH_3O$), 4.20-4.50 (1H; m, $CHCO_2Me$), 5.66-5.88 (1H, m, NH), 7.53 (5H, s, ArH), 7.70-8.20 (2H, br, $NH_3$). $R_f$(IV) = 0.74.

Anal. Calce. for $C_{19}H_{25}F_3N_2O_5$: C, 54.54; H, 6.02; N, 6.70. Found: C, 54.61; H, 6.05; N, 6.66.

This compound has utility as an intermediate in synthesis of an enkephalin peptide which inhibits muscle contraction.

Example XXIII

Cbz(1S)-$\nabla^E$Phe-Leu •OMe (23).

A solution of Cbz(1S)$\nabla^E$Phe (1.24 g, 4 mmol) and Leu ·OMe ·HCl (1.09 g, 6 mmol) in the THF (50 ml) was chilled to 0°C and triethylamine (0.61 g, 6 mmol), HOBt (0.54 g, 4 mmol) and DCC (0.83 g, 4 mmol) and DCC (0.83 g, 4 mmol) were added successively at 0° with stirring. After stirring for 4 hr at 0°C and room temperature overnight, the precipitated crystals were filtered and the filtrate was evaporated in vacuo. The residue was extracted with ethyl acetate and the extract was washed with 5% citric acid, 5% $\overline{NaHCO_3}$ and water successively, and then dried over $Na_2SO_4$. The solvent was removed under reduced pressure and the resulting solid was recrystallized from ethyl acetatehexane to give Cbz(1S)-$\nabla^E$Phe-Leu •OMe (23) (1.48 g, 84.5%) as a colorless needles, mp 114-115°C; $[\alpha]^{25}$-132.1° (c=1.0, MeOH). NMR ($CDCl_3$) δ : 0.78 6H, d, J = 6 Hz, $(CH_3)_2CH$), 1.10-1.70 (4H, m, CH-$CH_2$ and H), 2.18 (1H, d of d, J = 9 Hz, and 6 Hz, ∇H), 2.80 (1H, t, J = 9 Hz, $Ph^H$ Δ), 3.56 (3H, s, $CH_3O$), 4.17-4.45 (1H, m, $CHCO_2Me$), 5.25 (2H, s, $PhCH_2O$), 5.56-5.80 (1H, br, NH), 6.60-6.95 (1H, br, NH), 7.33 (5H, br s, Ph ∇), 7.47 (5H, s, $PhCH_2$). $R_f$(I) = 0.85, $R_f$(IV) = 0.12.

Anal. Calcd. for $C_{25}H_{30}N_2O_5$: C, 68.471 H, 6.90; N, 6.39. Found: C, 68.53; H, 6.93; N, 6.35.

This compound has utility as an intermediate in synthesis of an enkephalin peptide which inhibits muscle contraction.

Example XXIV

Cbz(1S)-$\nabla^E$Phe-Leu •OMe (24).

Following the same procedure as above for Cbz(1S)-$\nabla^E$Phe-Leu •OMe (23), Z-(2R)-$\nabla^E$Phe (1.24 g, 4 mmol) and Leu •OMe •HCl (1.09 g, 6 mmol) was treated with $Et_3N$ (0.61 g, 6 mmol), HOBt (0.54 g, 4 mmol) and DCC (0.83 g, 4 mmol) in THF (50 ml) to give Cbz(1S)-$\nabla^E$Phe-Leu •OMe (24) 1.41 g, 80.5%) as prisms (ethyl acetate-hexane): $[\alpha]_D^{25}$ 87.6° (C=1.0, MeOH); NMR ($CDCl_3$): 0.57 (3H, d, J = 6 Hz, $CH_3$), 0.68 (3H, d, J = 6 Hz, $CH_3$), 0.70-1.45 (4H, m, $CH_2CH$, H), 2.25 (1H, d of d, J = 9 Hz and 6 Hz, H), 2.77 (1H, t, J = 9 Hz 3,65 (3H, s, $CH_3O$), 4.15-4.50 (1H, m, $\overline{C}HCO_2Me$), 5.27 (2H, s, $PhCH_2O$), 5.73 (1H, s, NH), 6.83-7.15 (1H, br, NH), 7.32 (5H, br s Ph-CH-), 7.55 (5H, s, $PhCH_2$). $P_f$(I) = 0.85, $R_f$(III) = 0.12.

Anal. Calcd. for $C_{25}H_{30}N_2O_5$: C, 68.47; H, 6.90; N, 6.39. Found: C, 68.30; H, 6.96; N, 6.32.

Example XXV

(1R)-$\nabla^E$Phe-Leu •OMe •TFA (25).

Following a procedure similar to that above for (1S)-$\nabla^E$Phe-Leu •OMe •TFA (22), Z(1R)$\nabla^E$Phe (1.31 g, 4 mmol) was treated with thioanisole (2 ml) and TFA (20 ml) to give (1R)-$\nabla^E$Phe-Leu •OMe •TFA (25) (1.09 g, 87%), mp 256-257°C (dec.); $[\alpha]_D^{22}$ 24,2°, NMR ($CDCl_3$-$CF_3CO_2H$ (1:1)) δ : 0.83 (6H, d, J = 4 Hz, $CH_3$), 1.05-1.53 (3H, m, $CH_2CH$), 2.03-2.45 (2H, n, 3.53 (1H, t, J = 10 Hz, 3.75 (3H, s, $CH_3O$), 4.23-4.52 (1H, m, $CHCO_2Me$), 5.80 (1H, d, J = 8 Hz, NH), 7.52 (5H, s, ArH), 7.80-8.20 (2H, br NH). $R_f$(IV) = 0.77, $R_f$(V) = 0.80.

Anal. Calcd. for $C_{19}H_{25}F_3N_2O_5$: C, 54.54, H, 6.02; N, 6.70. Found: C, 54.56; H, 6.06; N, 6.66.

This compound has utility as an intermediate in synthesis of an enkephalin peptide which inhibits muscle contraction.

Example XXVI

CbzD-Ala-Gly-OMe (26).

A solution of CbzD-Ala (2.23 g, 10 mmol) and Gly •OMe •HCl (1.26 g, 10 mmol) in THF (50 ml) was chilled to 0°C and triethylamine (1.01 g, 10 mmol), HOBt (1.35 g, 10 mmol) and DCC (2.06 f, 10 mmol) was added successively at 0±C with stirring. After stirring for 4 hrs at 0°C, the reaction mixture was stirred at room temperature overnight and the precipitted crystals were filtered and the filtrate was evaporated in vacuo. The residue was extracted with ethyl acetate and the extract was washed with 5% citric acid, 5% $NaHCO_3$ and water successively, and dried over anhyd. $Na_2SO_4$. The solvent was removed in vacuo and the resulting solid was recrystallized from ethyl acetate-hexane to give Cbz-D-Ala-Gly-OMe (16) (2.48 g, 84.4%) as colorless needles, mp 96-97°C; $[\alpha]_D^{22}$ 23.3° (c = 1.0, MeOH): NMR ($CDCl_3$) δ : 1.39 (3H, d, J = 8 Hz, $CH_3$), 3.76 (3H, s $CH_3O$), 4.03 (2H, d, J = 6 Hz, $CH_2NH$), 4.15-4.50 (1H, m, -CH-NH), 5.15 (2H, s, $CH_2O$), 5.58 (1H, d, J = 7 Hz, NH), 6.70-6.95 (1H, br, NH), 7.42 (5H, s, Ar-H). $R_f$(I) = 0.62, $R_f$(VI) = 0.38.

Anal. Calcd. for $C_{14}H_{18}N_2O_5$: C, 57.14; H, 6.16; N, 9.52. Found: C, 57.19; H, 6.210; N, 9.50.

This compound has utility as an intermediate in synthesis of an analgetic peptide which inhibits muscle contraction.

Example XXVII

Cbz-Try-D-Ala-Gly-OMe (27).

A suspension of Cbz-D-Ala-Gly-OMe (26)(5.88 g, 0.02 mmol) and 10% Pd-C (0.4 g) in methanol (300 ml) was stirred under a hydrogen atmosphere at room temperature for 1.5 hr. The catalyst was fintered and the filtrate was evaporated in vacuo. The residue and Cbz Tyr (6.30 g, 0.02 mol) was dissolved in dry THF (300 ml), cooled to 0°C and HOBt (2.70 g, 0.02 mol) and DCC (4.12 g. 0.02 mol) were added successively at 0°C. The solution was stirred for 3 hr at 0°C and overnight at room temperature. The precipitated crystals were filtered. The filtrate was evaporated under reduced pressure, the residual crystals were dissolved in EtOAc and the solution was washed with 5% $NaHCO_3$, 0.2 N Hcl and water, and dried over anhyd. $Na_2SO_4$. The solvent wamoved in vacuo and the resulting solid was purified by silica gel column chromatography using $CHCl_3$-$CH_3OH$ (98:2) as eluant to give Cbz-Tyr-D-Ala-Gly-OMe (17) (8.62 g, 94.3%), mp 154-155°C (AcOEt-hexane); $[\alpha]_D^{22}$ 33.4° (c = 1.0, MeOH); NMR ($CDCl_3$-DMSO-$d_6$ (4:1) δ: 1.25 (3H, d, J = 7 Hz, $CH_3$), 2.82-3.03 (2H, m, $CH_2$,), 3.70 (3H, s, $CH_3O$), 3.80-3.95 (2H, m, $CH_2PhOH$), 4.15-4.60 (2H, m, 2 CH), 5.05 (2H, s, $CH_2O$), 6.70-7.25 (4H, m, ArH), 6.70-6.90 (1H, br, NH), 7.36 (5H, s, ArH), 7.70-8.10 (2H, br, NH). $R_f$(I) = 0.48; $R_f$(VI) = 0.08.

Anal. Calcd for $C_{23}H_{27}N_3O_7$: C, 60.39; H, 5.95; N, 9.18. Found: C, 60.23; H, 6.00; N, 9.08.

This compound has utility as an intermediate in synthesis of an analgetic peptide which inhibits muscle contraction.

Example XXVIII

Cbz-Tyr-D-Ala-Gly.OH (28).

To a solution of Cbz-Tyr-D-Ala-Gly-OMe (27) (4.57 g, 0.01 mol) in MeOH (10 ml) was added 1 N NaOH (20 ml, 0.02 mol) at 0° was stirring. The suspension was stirred for 2 hr at 0°C, diluted with water (80 ml), and neutralized with 1 N HCl (20 ml). The precipitated crystals were collected by suction, washed with water and dried under reduced pressure to give Cbz-Tyr-D-Ala-Gly.OH (18) (3.59 g, 81.0%), mp 102-104°C (AcOEt) (lit.[†] mp 1241C); $[\alpha]_D^{22}$ 18.0° (C = 1.0, DMF, (lit.[†] $[\alpha]_D^{EE}$ 16.7° (c = 0.54, DMF)); NMR (DMSO-$D_6$) δ: 1.16 (3H, d, J = 7 Hz, $CH_3$), 2.60-2.90 (2H, m, $CH_2$), 3.80 (2H, d, J = 6 Hz, $CH_2PHOH$), 4.10-4.45 (2H, m, 2CH), 5.02 (2H, s, $CH_2O$), 6.63-7.20 (4H, m, HOPh-), 7.40 (5H, s, ArH), 8.10-8.30 (2H, br, 2 NH), 9.10-9.40 (1H, br, OH). $R_f$(IV) = 0.20.

Anal. Calcd. for $C_{22}H_{25}N_3O_7$: C, 59.59; H, 5.68; N, 9.48. Found: C, 58.32; H, 5.79; N, 9.39. [†]S. Shinagawa, M. Fujini, H. Ishii and K. Kawai, Chem. Pharm. B ull., 29, 3630 (1981).

This compound has utility as an intermediate in synthesis of an analgetic peptide which inhibits muscle contraction.

Example XXIX

Cbz-Tyr-D-Ala-Gly(1S)-$\nabla^E$Phe-Leu •OMe (29).

To a solution of Cbz-Tyr-D-Ala-Gly • OH (887 mg, 2 mmol) and (2S)-$\nabla^E$Phe-Leu •TFA (836 mg, 2 mmol) in THF (80 ml) was added N-methylmorpholine (202 mg, 2 mmol) HOBt (270 mg, 2 mmol) and EDC

(1-ethyl-3(3-dimethylaminopropyl)-carbodimide• HCl) (384 mg, 2 mmol) successively at 0°C. After stirring for 3 hr at 0°C and overnight at room temperature, the solvent was removed under reduced pressure, and the residue was extracted three with 100 ml of AcOEt. The combined AcOEt extracts were washed with 5% $NaHCO_3$, 0.2 N HCl, water, and dried over anhyd. $Na_2SO_4$. The solvent was removed under reduced pressure, and the solid was recrystallized from ethylacetate-hexane to give 1.212 g (83%) of Cbz-Tyr-D-Ala-Gly(1S)- $\nabla^E$Phe-Leu •OMe (29) as a colorless powder, mp 162-163°C; $[\alpha]_D$-56.8° (c = 0.5, DMF). NMR (DMSO-$d_6$) wδ: 0.78 (6H, t, J = 5 Hz, $CH_3$x2), 1.00-1.67 (4H, m, $CH_2$CH and cyclopropyl-H), 1.18 (3H, d, J = 7 Hz, $CH_3$), 1.90-2.15 (1H, m, cyclopropyl-H), 2.46-3.00 (3H, m, $CH_2$ and cyclopropyl-H), 3.48 (3H, s, $CH_3$O), 3.70-3.80 (2H, br, $CH_2$) 3.95-4.43 (3H, m, 3 CH), 5.01 (2H, s, $CH_2$), 6.67-7.18 (4H, m, HOPh-), 7.32 (5H, s, ArH), 7.40 (5H, s, Ar-H), 7.30-7.60 (1H, m, NH), 7.72 (1H, d, J = 8 Hz, NH), 8.10-8.40 (2H, m, 2 NH), 8.68 (1H, s, NH), 9.28 (1H, s, OH). $R_f$(II) = 0.79; $R_f$(IV) = 0.63.

Anal. Calcd, for $C_{39}H_{47}N_5O_9$: C, 64.18; H, 6.49; N, 9.60. Found: C, 64.04; H, 6.54; N, 9.56.

This compound has utility as an intermediate in synthesis of an analgetic peptide which inhibits muscle contraction.

Example XXX

Cbz-Tyr-D-Ala-Gly-(1R)-$\nabla^E$Phe-Leu •OMe (30).

Following a procedure similar to that described for Cbz-Tyr-D-Ala-Gly (2S)-$\nabla^E$Phe-Leu •OMe (29), Cbz-Tyr-D-Ala-Gly •OH (887 mg, 2 mmol); (R)-$\nabla^E$Phe-Leu •OMe •TFA (836 mg, 2 mmol), N-methylmorpholine (202 mg, 2 mmol), HOBt (270 mg, 2 mmol), EDC (384 mg, 2 mmol), and THF (80 ml) gave 1.287 g (88.2%) of Cbz-Tyr-D-Ala-Gly-(1R)-$\nabla^E$Phe-Leu •OMe (30) as a colorless powder, mp 160-170°C (AcEOt-hexane). NMR (DMSO-$d_6$) δ: 0.53 (3H, d, J = 5 Hz, $CH_3$), 0.73 (3H, d, J = 5 Hz, $CH_3$), 1.20 (3H, d, J = 6 Hz, $CH_3$), 1.05-1.45 (4H, m, $CH_2$CH and ∇H), 1.87-2.13 (1H, m, ∇H), 2.43-2.60 (1H, m, ∇H), 2.60-2.90 (3H, m, $CH_3$), 3H, s, $CH_3$O), 3.70-3.85 (2H, br, $CH_2$), 3.90-4.45 (3H, m, CHx3), 5.03 (2H, s, $CH_2$), 6.66-7.23 (4H, m, HOPh)-7.31 (5H, s, ArH), 7.40 (5H, s, ArH), 7.40-7.85 (2H, m, NHx2), 8.15-8.40 (2H, br, NHx2), 8.70 (1H, s, NH), 9.25 (1H, s, OH). $R_f$(II) = 0.81, $R_f$(IV) 0.63.

Anal. Calcd, for $C_{39}H_{47}N_5O_9$: C, 64.18; H, 6.49; N, 9.60. Foyund: C, 63.99; H, 6.51; N, 9.56.

This compound has utility as an intermediate in synthesis of an analgetic peptide which inhibits muscle contraction.

Example XXXI

Cbz-Tyr-D-Ala-Gly-(25)$\nabla^E$Phe-Leu •OH (31).

Cbz-Tyr-D-Ala-Gly-(1S)$\nabla^E$Phe-Leu •OMe (29) (365 mg, 0.5 mmol) was dissolved in methanol (1 ml), the solution was cooled in an ice-water bath and 1N NaOH (1 ml, 1 mmol) at 0°C was added. After stirring for 2 hr at 0°C, the solution was neutralized with 1N HCl and diluted with water. The precipitated solid was collected by suction and dried under reduced pressure. The solid was purified by silica gel column chromatography using chlorofoirm-methanol (19:1) and chloroform-methanol-aceit acid (95:5:1) as eluants. After starting material was removed using $CHCl_3$/MeOH (19:1), Cbz-Tyr-D-Ala-Gly-(1S) $\nabla^E$Phe-Leu •OH (31) 230 mg (64%), mp 153-155°C (AcOEt). Intermediate to Example XXXIV. $R_f$(IV) = 0.48; Rf(V) = 0.88; $[\alpha]_D^{22}$ -98.2° (c.o.5, DMF), was eluded with $CHCl_3$:MeOH:HOAc (95:5:1).

Anal. Calcd. for $C_{38}H_{45}N_5O_9.H_2O$: C, 62.20; H, 6.46; N, 9.54. Found: C, 62.39; H, 6.50, N, 9.30.

Example XXXII

Cbz-Tyr-D-Ala-Gly-(1R)$\nabla^E$Phe-Leu •OH (32).

According to a procedure similar to that described above, Cbz-Tyr-D-Ala-Gly-(1R)$\nabla^E$Phe-Leu •OMe (30) (365 mg, 0.5 mmol) gave 270 mg (75%) of Cbz-Tyr-D-Ala-Gly-(1R)$\nabla^E$Phe-Leu •OH (32), mp 204-205°C. (AcOEt); Rf(IV) = 0.40; Rf(V) = 0.80; $(\alpha)_D^{22}$ -41.2° (c.0.5, DMF).

Anal. Calcd. for $C_{38}H_{45}N_5H_2O$: C, 62.20; H, 6.46, N. 9.54. Found: C, 62.28; H, 6.46; N, 9.38.

Example XXXIII

Tyr-D-Ala-Gly-(1S)$\nabla^E$Phe-Leu (33).

A solution of Z-Tyr-D-Ala-Gly-(1S)$\nabla^E$Phe-Leu •OH (31) (143 mg, 0.2 mmol) and thioanisole (0.3 ml) in trifluoroacetic acid (TFA) (3 ml) was stirred 1 hr at 0°C, and 4 hr at room temperature. The solvent was removed under reduced pressure and the residue was triturated with ether to give a TFA salt. This salt was dissolved in 5% AcOH and passed through a column containing a large excess of Amberlite CG400 (acetate form), followed by a column of BioGel P-2 (1.9x8.9 cm, 200 400 mesh) (4ml/20 min). The fractions containing Tyr-D-Ala-Gly-(1S) $\nabla^E$Phe-Leu (33) were pooled and lyophilized to give 70 mg (60.1%) of (S)-10, mp 197°C (dec.); ret. time = 4.4 min (HPLC, $C_{18}$-Lichrosorb (20 cm x 0.46 cm), $CH_3CN$: $H_2O$:TFA (30:70:1), 1 ml/min; $R_f$(IV) = 0.05; $R_f$(VI)0.38 $[\alpha]_D^{22}$ -7.12° (c, o.25 AcOH). Amino acid ratios in acid hydrolyzate: Tyr .94; Ala 0.92; Gly 1.0; Leu 1.01; [(1S)$\nabla^E$Phe was not detected].

Anal. Calcd. for $C_{36}H_{39}N_5O_7.0.8H_2O$: C, 60.45; H, 6.86; N, 11.74. Found: C, 60.62; H, 6.89; N, 11.42.

This compound has utility as a final peptide in synthesis of an analgetic peptide which inhibits muscle contraction.

Example XXXIV

Tyr-D-Ala-Gly-(1R)$\nabla^E$Phe-Leu (34).

Following a procedure similar to that described above, Z-Tyr-D-Ala-Gly-(1R)$\nabla^E$Phe-Leu •OH (32) (0.43 mg, 0.2 mmol) gave 82 mg (70.4%) of (2R)-10, mp 185°C (dec.); ret. time 8.1 min [HPLC, $C_{18}$-Lichrosorb (20 cm x 0.46 cm), $CH_3CN$:$H_2O$:TFA (30:70:1), 1 ml/min]; Rf(IV) = 0.03; Rf(V) = 0.35; $[\alpha]_D^{22}$ 89.6 (c 0.25, AcOH). Amino acid ratios in acid hydrolazate: Tyr 0.91, Ala 0.98, Gly 1.0, Leu 0.95. [(1SR)$\nabla^E$Phe was not detected].

Anal. Calcd. for $C_{30}H_{39}N_5O_7.CH_3CO_2H$: 1.5 $H_2O$; C, 57.47; H, 6.93; N, 10.47. Found: C, 57.56; H, 6.67; N, 10.08.

This peptide is an active analgetic agent also.

These new peptides are extremely refractory towards hydrolysis. The cyclopropane ring of the variant amino acid sterically hinders reactions at the adjacent carbonyl and amino functions and the $\beta$-groups $CO_2H$ and phenyl will retard the enzyme splitting or cleavage of the peptide linkages.

Short chain dipeptides where both amino acids have been cyclopropylized will be particularly stable to acid hydrolysis at the methylester group and the peptide linkage both. Unless the peptides themselves are toxic to humans no added toxicity due to the modified unnatural amino acid components should be exhibited by the sweetener.

The following example illustrates a specific end product embodiment within the scope of this invention and is not to be construed as limiting said scope. Where the invention has been described herein with regard to a certain specific embodiment, it is not so limited. It is to be understood that variations and modifications thereof may be made by those skilled in the art without departing from the scope of the invention.

It must be remembered that the sweetening properties of the aspartame type of peptide is dependent upon the stereochemistry of the individual amino acid components of the peptide. Each of the amino acids can exist in either dextro (D) or leavo (L) rotatory form. The L form of the compounds possess the sweetness characteristic as best can be determined at this point. The D form of isomers are usually bitter or tastless. Combinations of the D and L forms of isomers are sometimes sweet also but usually not as intense in their sweetness as the L-isomers.

The new dipeptide containing 1-aminocyclopropane-1-carboxylic acid derivatives of the following example are water soluble substances which can be used in a number of applications where a non toxic non sugar type sweetener is desired. They are l00-200 times as sweet as sucrose and exhibit long term retention of that sweetening capacity. Moreover, the new dipeptides do not exhibit the unpleasant aftertaste characteristic of saccharine and cyclamates.

These dipeptide compositions are particularly valuable as sweetening agents for carbonated and noncarbonated beverages, chewing gum edible materials such as fruit juices, vegetables, fruit, dairy products such as egg product, milk drinks, ice cream, syrups, cake mixes, instant beverage mixes, soda pop formulae, iceing and dessert toppings, sherbert, meat products, wines and salad mixes, and dressings.

Example XXXV

N-CBZ-$\beta$-Benzyl-L-aspartyl-$\nabla^Z$-phenylalanine methyl ester (27)

A total of l.57 g (4.l mmoles) of N-CBZ-aspartic acid-$\beta$-benzyl ester dissolved in 50 ml of dry THF was

cooled to -20°C in a Dry Ice-carbon tetrachloride bath and 0.58 ml (5.3 mmoles) of N-methylmorpholine and 0.68 ml (5.3 mmoles) of isobutyl chloroformate were added. After 20 minutes a solution of 1.0 g (4.4 mmoles) of 25 in 20 ml of dioxane-water (7:3) containing 0.61 ml (4.4 mmoles) of triethylamine was added. The mixture was allowed to stir at room temperature overnight, the THF was removed in vacuo, 50 ml of ether and 10 ml of $H_2O$ were added and the solution was extracted. The aqueous portion was extracted with additional ether (2 x 25 ml), the ether layers combined, washed with 5% citric acid (2 x 25 ml), 5% $NaHCO_3$ (2 x 25 ml), saturated NaCl (1 x 2 ml), and dried over anhydrous $Na_2SO_4$. The solution was filtered, concentrated to ca 25 ml and hexanes added until cloudy. Crystallization at room temperature gave 1.73 g of 27 as a white crystalline solid. The filtrate was stored at 5°C to affored an additional 0.22 g of 27 for a total yield of 84%, m.p. 99-104°; $R_f$(IV) = 0.80; IR (KBr) 3310 (amide NH), 1750-1650 $cm^{-1}$ (CBZ C=O, ester C=O, amide C=O); NMR ($CDl_3$) δ 7.5-7.3 (m, 15H, ArH), 6.5 (br, s, 1H, NH), 5.9-5.7 (m, 1H, NH), 5.2-5.0 (dd, 4H, $PhCH_2OCO$-), 4.5 (, 1H, α-H), 3.7 (2s, 3H, diastereomeric $COOCH_3$), 3.1-2.9 (, 1H, ), 2.8-2.6 (, 2H, COO-$CH_2$-), 2.3-2.1 and 1.9-1.8 (m, 2H, ).

Anal. Calcd. for $C_{30}H_{30}O_7N_2$: C, 67.92; H, 5.66; H, 5.28. Found: C, 67.98, H, 5.73; N, 5.30.

L-Aspartyl-$\nabla^Z$-phenylalanine methyl ester (29).

A total of 1.0 g (1.9 mmoles) of 27 was dissolved in 50 ml of absolute methanol, 70 mg of 10% Pd/C was added, and hydrogen bubbled through the solution for 5 hours. The solution was filtered through celite, and the filtrate concentrated in vacuo. Partition chromatography of this material on Sephadex G-10 using n-BuOH/HOAc/$H_2O$ 4:1:5 upper phase as eluant afforded one pure diastereomer of 29 (now termed 29A) in fractions 41-90, the other pure diastereomer in fractions 112-150 (now termed 29B), and a mixture of the two diastereomers in fractions 91-111. Fractions 41-90 were combined, concentrated in vacuo, 40 ml of 5% acetic acid was added, and the solution was lyophilized to give 150 mg of 29A. Fractions 91-111 were treated similarly to give 147 mg of 29A and 29B. Fractions 112-150 were treated similarly to give 210 mg of 29B, for a total yield of 88%, m.p. 29B 126-180°¶; $[\alpha]_D^{25}$ 29A=35° (c.0.7, in 1N Hcl), $[\alpha]_D^{25}$ 29B = 1.3° (c 1.1, in 1N HCl); $R_f$ =29A (V) = 0.36, $R_f$ 29B (V) = 0.30; NMR ($CD_3OD$) 29A and 29B δ 7.5-7.4 (br s, 5H, ArH), 5.3 (29B) and 5.1 (29A) (amide NH), 4.6-4.2 (m, 1H, α-H), 3.8 (2s, 3H, $COOCH_3$), 3.2-3.0 (m, 1H, AL-OH), 2.6-2.4 (m, 2H, OOC-$CH_2$-), 2.3-1.8 (m, 2H, cp-$CH_2$)

This, peptide is a potent long term sweetener for carbonated beverages when used in a dose of 373 milligrams per 12 ounce can of carbonated orange soda, or cola beverage.

Anal. Calcd. for $C_{15}H_{18}O_5N_2$.1 $H_2O$ (29B): C, 55.5; H, 6.17; N, 8.64. Found: C, 55.71; H, 5.92; N, 8.38.

Example XXXVI

N-CBZ-β-Benzyl-L-aspartyl-$\nabla^E$phenylalanine methyl ester (28).

A total of 0.86 g (2.4 mmoles) of N-CBZ-L-aspartic acid-β-benzyl ester dissolved in 30 ml of dry THF was cooled to 20° in a Dry Ice-carbon tetrachloride bath and 0.32 ml (2.9 mmoles) of N-methylmorpholine and 0.38 ml (2.9 mmoles) of isobutyl chloroformate were added. After 20 minutes a solution of 0.55 g (2.4 mmoles) of 26 in 10 ml of dioxane-water (7:3) containing 0.33 ml (2.4 mmoles) of triethylamine was containing 0.33 ml (2.4 mmoles) of triethylamine was added. The mixture was allowed to stir at room temperature overnight, the THF was removed in vacuo, 30 ml of ether and 10 ml of $H_2O$ were added and the solution was extracted. The aqueous portion was extracted with additional ether (2 x 10 ml), the ether layers combined, washed with 5% citric acid (2 x 20 ml), 5% $NaHCO_3$ (2 x 20 ml), water (1 x 20 ml), and dried over anhydrous $Na_2O_4$. The solution was filtered, concentrated in vacuo, and the resultant oil dried in vacuo over $P_2O_5$ to given 1.1 g (86%) of 28 as an amorphous solid. $R_f$(V)) = -.38; NMR ($CDCl_3$) δ 7.5-7.4 (br s, 4H, $PhCH_2OCO$-), 4.8-4.6 (m, 1H, α-H), 3.4 (br s, 3H, $COOH_3$), 3.1-3.7 (m, 3H, and COO-$CH_2$), 2.4-2.1 and 1.6-1.4 (m, 2H, cp-$CH_2$).

Thus, compound is an intermediate for XXXVII.

Example XXXVII

L-Aspartyl-$\nabla^E$-phenylalanine methyl ester (30). .

A total of 1.5 g (2.8 mmoles) 28 was dissolved in 50 ml of absolute methanol, 100 mg of 10% Pd/C was added, and hydrogen was bubbled through the solution for 4 hours. The solution was filtered through celite and the filtrate concentrated in vacuo. Partition chromatography of this material on Sephadex -10 using n-BuOH/HOAc/$H_2O$ 4:1:5 upper phase as eluant afforded pure 30 in fractions 94-107. These fractions were combined, the solvent removed in vacuo, and the resultant oil precipitated from methanol/ether to give 140 mg (16%) of 30, $R_f(V)$ = 0.25; NMR ($CD_3OD$) δ 7.6-7.3 (m, 5H, arH), 4.5-4.1 (m, 1H, α-H),3.4 (s, 3H, $COOH_3$), 3.1-3.9 (m, 1H, ), 2.5-2.3 (m, 2H, OOC-$CH_2$-), 2.3-1.7

This peptide is a potent long term sweetener for beverages and food products.

Example XXXVIII

Synthesis of 1-aminocyclopropyl-2-carboxylic Acid-1-Carboxyl Phenylalanine Methyl Ester

a. N-Boc-Dehydro-Aspartic Acid β-t-Butyl α-Methyl Ester

A solution of 5 g of N-Boc-β-hydroxyaspartic acid ester in 25 mL of acetic anhydride should be heated to a temperature of app. l00° for l hr. and is evaporated to dryness in vacuo. The crude dehydro aspartic acid derivative should be precipitated from an appropriate solvent such as ethyl acetate using petroleum ether and used directly in the next step. The yield is quantitative. This is first intermediate.

b.N-Boc-1-aminocyclopropane-1,2-dicarboxylic acid 0-2-t-Butyl Ester-0-1-Methyl Ester

The crude dehydro aspartic acid derivative should be dissolved in 50 mL of methylene chloride and gaseous diazomethane passed into the solution until excess reagent is present in the solution. After standing overnight, the solution is evaporated and the residue is dissolved in l0 mL of dry benzene and refluxed until no further $N_2$ is evolved. Evaporation of the solution gives the crude product which is purified by crystallization from an appropriate solvent or chromatographed on a silica column. If E- and Z-isomers are present these are to be separated by careful chromatography.

c.N-Boc-1-aminocyclopropane-1,2-dicarboxylic Acid 0-2-t Butyl Ester

To a solution of 5 g of N-Boc-VAsp(0tBu)0Me in 50 mL of methanol should be added a 1.1 molar excess of 4N NaOH and the solution is allowed to stand at room temperature for four hours. The solution should then be evaporated to a very small volume in vacuo, diluted with 50 mL of water, and ten percent citric acid solution added until the pH is 5-6. The product precipitates and is collected on a filter. After washing with water, the product is dried in a dessiccator and is crystallized from ethyl acetate. Yield quantitative.

d.N-Boc-0t Butyl-1-Aminocyclopropane-2-carboxylic Acid-1-carboxyl Phenylalanine Methyl Ester

To a solution of 5 g of N-Boc-1-aminocyclopropane-1,2-dicarboxylic acid 0-2-t butyl ester in 50 mL of methylene chloride should be added 1 molar equivalent each of dicyclohexyl carbodiimide, N-hydroxybenztriazole, and phenylalanine methyl ester. After 16 hr. at room temperature, the mixture should be filtered and the filtrate washed successively with 10% $NaHCO_3$, 10% HCl and brine and dried over anhyd. $Na_2SO_4$. After evaporation to dryness, the residue should be crystallized from an appropriate solvent such as ethyl acetate. Yield is 70%. 1-Aminocyclopropane-2-carboxylic Acid-1-carboxyl Phenylalanine Methyl Ester

To a 1:1 solution of trifluoroacetic acid and methylene chloride (50 mL) should be added 5 g of N-Boc β-t-butyl cyclopropyl aspartyl phenylalanine methyl ester and the solution is allowed to stand at room temperature for one hour and evaporated to room temperature f or one hour and evaporated to dryness. The crude peptide should be dissolved in 10% acetic acid and the solution should be passed through a column of Biogel P-2 and the ninhydrin reactive fractions pooled and lyophilized. Crystallization of the pure peptide from aqueous alcohol or some appropriate solvent mixture gives pure cyclopropyl aspartyl phenylalanine methyl ester, yield quantitative. This is the final product.

This peptide is very sweet to the taste and can be used to sweeten soft drinks, foods of all kinds, etc.

As noted above the new sweetner compounds have about l00 times to 200 times the sweetness power of ordinary sucrose. To sweeten an 227g (8 ounce) cup of coffee a l gram quantity of the new product can be employed for example depending on the taste of the user. One packet of 0.035 oz. or l gram of the products of Examples XXXV or XXXVIII is equivalent in sweetness power to 2 teaspoonfuls of sucrose.

A significant advantage of the present product of Examples XXXV and XXXVIII is that they do not hydrolyze in the intestine of the consumer when contacted by intestinal enzymes such as carboxypeptidase and chymotrypsin to generate methanol which leads to formation of formaldehyde. This means that a user can drink a cup of coffee, soda or other beverage sweetened with the cyclopropylized dipeptide ∇Asp-Phe-$OCH_3$ or ASP-∇PHE.$OCH_3$ and while the sweetner performs its designated function it does not hydrolyze cleave or break down in the gastrointestinal tract or blood for at least 24 hours. This is sufficient time for the product in vivo to pass out of the body either with the feces or the urine.

The significance of this lack of hydrolysis is that it has been alleged that metabolic fragments, i.e., hydrolysis products of normal L-Asp-Phe •$OCH_3$ do cause in some consumers side effects such as behavior modification, hyperactivity genetic changes, and brain tumors. The following comparative in vitro study of commercial ASPARTAME and the new food sweeteners of Example XXXV is presented to demonstrate the unexpected and beneficial utility in synthetic sweetners exhibited by the product of the invention.

COMPARATIVE PEPTIDE TESTS FOR STABILITY

Aspartame and new stabilized Asp-$\nabla^Z$-Phe-OMe (29) of Example XXXV were comparison tested for stability to enzymatic hydrolysis by α-chymotrypsin. The product from hydrolysis of Aspartame would be Asp-Phe-OH (2), and from 29 Asp-$\nabla^Z$-Phe-OH. A ratio of 10:1 substrate:enzyme was used in a 0.5 M ammonium acetate solution which was adjusted to pH 8 with 10% ammonium hydroxide. After 15 minutes at room temperature aspartame was hydrolyzed to give the acid (2) and methanol. After 24 hours at room temperature, 29 failed to hydrolyze. Thus, response to hydrolysis by α-chymotrypsin of compound 29 is completely different than the response of aspartame. The new food product of Example XXXV is hence demonstrated to have a sharp and distinct new property of resistance to peptide degradation by hydrolysis.

Example XXXIX

A powdered sweetener mix for use as the equivalent of sucrose can be prepared by mixing:

| Ingredient | Grams |
|---|---|
| Dipeptide of Example XXXV or Example XXXVIII | 0.2l6 |
| Dextrose solids (50%) Corn syrup solids (50%) | l.70 |
| Tribasic Calcium Phosphate | 0.0l |
| Cellulose gum as a Microcrystalline cellulose | 0.ll |

This dry powderous material is free flowing and soluble instantly in aqueous hot or cold liquids. A single dose of l gram of this product will sweeten 300 c.c. of a carbonated or non carbonated aqueous beverage. It is stable against all types of hydrolyzing agents, including food acids.

The new peptide sweetener prepared by the methods of the preceding examples can be advantageously employed in a new and unique beverage composition. This new food composition has the added merit over ordinary aspartame sweetened beverages in that despite the presence of an organic acid in the formulation the composition has long term stability of its sweetener ingredients. The following is an illustration of such a practical use although it is clear that a number of other food applications are feasible.

Example XL

A fruit flavored beverage mix can be prepared employing the following formulation which includes the new peptide sweetener:

| Ingredient | Parts by Weight |
|---|---|
| Peptide Sweetener of Example XXXV | 0.89 |
| citric Acid | 5.53 |
| Clouding agent as shown in U.S. Patent 3,023,106 | 2.28 |
| Sodium carboxymethyl cellulose | 0.90 |
| Tricalcium phosphate | 0.49 |
| TRisodium citrate | 0.70 |
| Vitamin C | 0.47 |
| Tenfold orange oil | 0.26 |
| Vitamin A | 0.04 |
| Color (mixing 50/50 FDC No. 5 and FDC No. 6 - yellow) | 0.01 |

6.5 grams of the above beverage mix can be reconstituted in a pint of water to produce a beverage exhibiting a highly acceptable mouth feel, cloud stability and natural fruit juice appearance.

Example XLI

A beverage can be manufactured and placed in 12 ounce cans which has a high margin of freedom from peptide sweetner decomposition and attendant problems of generation of methanol. The composition comprises:

| Ingredient | Grams |
|---|---|
| Carbonated water, | 299 |
| Caramel color, | 0.1 |
| Phosphoric Acid, | 0.2 |
| Artificial flavor, | 0.12 |
| Preservative-Sodium Benzoate, | 0.2 |

| Ingredient | Grams |
|---|---|
| Citric acid, | 0.15 |
| Caffeine, and | 0.1 |
| Peptide of Example XXXV | 2.16 |

This makes 300 c.c. of carbonated soda.

PREFERRED EMBODIMENT-PHARMACEUTICAL APPLICATIONS-POLYPEPTIDES

The following Examples XLII and XLIII will serve to illustrate the preparation of several of our new peptides having enhanced stability against enzymatic cleavage as applied to the control of blood pressure. These peptides are renin inhibitors and are orally, intramuscularly or intravenously administered in dosage unit amounts comparable to those of known renin inhibitors. For instance, in a case where a peptide is formulated in a 50 cc vial it may contain a 50 milligram dose of peptide in glucose or isotonic saline solution for intravenous administration. The modified peptide of Examples XLII and XLIII will be substituted in like quantity and concentration whether the dose given is by oral or systemic administration to the subject.

SYNTHESIS OF RENIN INHIBITORS

Example XLII

Boc-∇Leu-Val-Tyr-OMe

To a solution of 2.43 g(10 mm) of Boc-∇Leu-OH in 50 ml of $CH_2Cl_2$ there is to be added 1.0 g (10 mm) of triethylamine and 1.4 g (10 mm) of isobutyl chloroformate at 0°. After 15 min. a solution of 2.94 g (10

mm) Val-Tyr-OMe in the same solvent is to be added and the mixture is stirred one hour at 0° and two hours at room temperature. After evaporation of the solution, the residue is to be dissolved in ethyl acetate and the solution should be washed successively with water (2 x 25 mL), 0.1 N HCl (2 x 25 mL), 5% $NaHCO_3$ (2 x 25 mL) and brine (2 x 25 mL). After drying over anhyd. $Na_2SO_3$ the solution is evaporated to dryness and the crude peptide is crystallized from ethyl acetate or a suitable solvent; yield 89%. This compound is a useful intermediate

∇Leu-Val-Tyr-OMe

To 25 ml of a 1:1 mixture of $CH_2Cl_2$ and trifluoroacetic acid is to be added 5.19 g (10 mm) of Boc-∇Leu-Val-Tyr-OMe and the solution should be allowed to stand one hour at room temperature. Evaporation of the solution gives the crude tripeptide as the TFA salt, ∇Leu-Val-Tyr-OMe-TFA, to be used directly in the next step.

Boc-Pro-Phe-His-Leu-∇Leu-Val-Tyr-OMe

To a solution of 6.26 g (10 mm) Boc-Pro-Phe-His-LeuNHNH$_2$ in 50 mL of dry DMF should be added 1.17 g (10 mm) of AmONO while cooling in an ice bath. After one hour should be added a DMF solution containing 5.33 g (10 mm) of ∇Leu-Val-Tyr-OMe-TFA and 1.01 g (10 mm) of triethylamine over a period of fifteen min. After one hour at 0-5° and five hours at room temperature, the solution is to be partitioned between 250 mL of water and 500 mL of ethyl acetate in a separatory funnel. The organic layer is separated and washed successively with water (3 x 50 mL), 0.1 N HCl (2 x 50 mL), 5% $NaHCO_3$ (2 x 25 mL) and brine (2 x 25 mL) and dried over anhyd. $Na_2SO_4$. Evaporation of the solution will give the crude blocked heptapeptide which is to be purified by crystallization from ethyl acetate or other suitable solvent. This is also an intermediate.

Boc-Pro-Phe-His-Leu-∇Leu-Val-Tyr-OH

To a solution fo 10.1 g (10 mm) of Boc-Pro-Phe-His-Leu- ∇Leu-Val-Tyr-OMe in 50 mL of methanol is to be added 5 mL of 2N NaOH at room temperature. After two hours, the solution is to be evaporated to dryness and the residue is to be dissolved in 25 mL of water. The solution is acidified to pH 4 with 10% citric acid and the precipitated peptide is filtered and washed with water. After drying the solid in vacuo, the solid is crystallized from water and ethanol or some other suitable solvent to give a 90% yield of Boc-Pro-Phe-His-Leu- ∇Leu-Val-Tyr-OH. . This is an intermediate.

Pro-Phe-His-Leu-∇Leu-Val-Tyr-OH

A solution of 9.99 g (10 mm) of Boc-Pro-Phe-His-Leu- ∇Leu-Val-Tyr-OH in 25 mL of 1:1 $CH_2Cl_2$/TFA is to be allowed to stand at room temperature for one hour. After evaporation to dryness, the residue is dissolved in 5% HOAc solution and passed through a Biogel P-2 column and the fractions reactive to ninhydrin are pooled and lyophilized: yield of pure Pro-Phe-His-Leu-∇Lei-Val-Tyr-OH is 90%.

This peptide should bind renin very tightly and be active as a renin inhibitor.

Example XLIII

Boc-∇Leu-Leu-Val-Tyr-OH

To a solution of 2.43 g (10 mm) of Boc-∇Leu-OH in 50 mL of THF in a 250 mL round bottomed flask should be added 1.0 g (10 mm) of triethylamine and 1.4 g (10 mm) of isobutyl chloroformate with ice cooling. After 15 min., 4.07 g (10 mm) of leu-Val-Tyr-OMe (10 mm) is to be added and the solution is stirred at 0-5° for one hour and room temperature overnight. After removal of the solvent in vacuo the residue is dissolved in 100 mL of ethyl acetate and washed successively with 0.1 N HCl (2 x 25 mL), 5% $NaHCO_3$ (2 x 25 mL) and saturated NaCl (2 x 25 mL). After drying over anhyd. $NaSO_4$, the solution should be evaporated to dryness in vacuo. The crude product is recrystallized from a suitable solvent like ethyl acetate to give the pure tripeptide, Boc-∇Leu-Leu-Val-Tyr-OMe, in 75% yield.

∇Leu-Leu-Val-Tyr-OMe.

To a 1:1 mixture of $CH_2Cl_2$ and trifluoroacetic acid is to be added 6.32 g (10 mm) of Boc-∇Leu-Leu-Val-Try-OMe and the solution should be allowed to stand one hour at room temperature. Evaporation of the solution will give the crude tetrapeptide TFA salt, ∇Leu-Leu-Val-Tyr-OMe-FTA, used without purification in the next step.

Example XLIV

Boc-Pro-Phe-His-∇Leu-Leu-Val-Tyr-OMe

To a solution of 5.13 g (10 mm) of Boc-Pro-Phe-HisNHNH$_2$ in 50 mL of dry DMF should be added 1.17 g (10 mm) of AmONO while cooling in an ice bath. After one hour is added a DMF solution containing 6.46 g (10 mm) of ∇Leu-Leu-Val-Tyr-OMe-TFA and 1.01 g (10 mm) of triethylamine over a period of fifteen min. After one hour at 0-5° and five hours at room temperature, the solution is to be partitioned between 250 mL of water and 500 mL of ethyl acetate in a separatory funnel. The organic layer is separated and washed successively with water (3 x 50 mL), 0.1N HCl (2 x 50 mL), 5% $NaHCO_3$ (2 x 50 mL) and brine (2 x 25 mL) and dried over anhyd. $Na_2SO_4$. Evaporation of the solution will give the crude blocked heptapeptide which is to be purified by recrystallization from ethyl acetate or some other suitable solvent. This is an intermediate.

Boc-Pro-Phe-His-∇Leu-Leu-Val-Tyr-OH

To a solution of 10.1 g (10 mm) of Boc-Pro-His-∇Leu-Leu-Val-Tyr-OMe in 50 mL of methanol is to be added 5 mL of 2N NaOH at room temperature. After two hours, the solution is evaporated to dryness in vacuo and the residue is dissolved in 25 mL of water. The solution is acidified to pH 4 with 10% citric acid and the precipitated peptide is to be filtered and washed with water. After drying over anhyd. $CaCL_2$ in vacuo the solid is to be recrystallized from ethanol and water to give a 90% yield of Boc-Pro-Phe-His-∇Leu-Leu-Val-Tyr-OH. . This is an intermediate.

Pro-Phe-His-∇Leu-Leu-Val-Tyr-OH

A solution of 9.99 g (10 mm) Boc-Pro-Phe-His-∇Leu-Leu-Val-Tyr-OH in 25 mL of 1:1 $CH_2Cl_2$/TFA is to be allowed to stand at room temperature for one hour. After evaporation ofthe solution, the residue is passed through a Biogel P-2 column in 5% HOAc solution and the fractions reactive to ninhydrin are to be pooled and lyophilized; yield of pure Pro-Phe-His-∇Leu-Leu-Val-Tyr is 90%.

This peptide should bind renin very tightly and be active as a renin inhibitor.

**Claims**
**Claims for the following Contracting States : BE, CH, DE, FR, GB, IT LI, LU, NL, SE**

**1.** A 1-aminocyclopropane-1-carboxylic acid selected from the group consisting of (1S)-E-, (1R)-E, (1S)-Z, (1R)-Z-, (1S)-, (1R)-, (1RS)-E-, and (1RS)-Z-isomers with respect to the carbon in the 1-position, having the formula

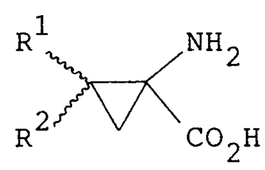

wherein $R^1$ and $R^2$ are, in either order, a pair of substituents selected from the group consisting of $-CH_3$ and $-CH_3$, $-CH(CH_3)_2$ and -H, $-CO_2H$ and -H, $-CH_2CO_2H$ and -H, $-CH_2SCH_3$ and -H, $-(CH_2)_3NH_2$ and -H, $-CH_2CH_2NHC(=NH)NH_2$ and -H, $-(CH_2)_2NH_2$ and -H, 3,4-dihydroxyphenyl and -H, 3-indolyl and -H, 5-hydroxy-3-indolyl and -H, -OH and -H, -SH and -H, $-CH_2SH$ and -H, $-CH_2OH$ and -H, and 4(5)-imidazolyl and -H.

**2.** A heterocyclic cyclopropane-1-carboxylic acid selected from the group consisting of (1S)- and (1R)-isomers with respect to the carbon in the 1-position, having the formula

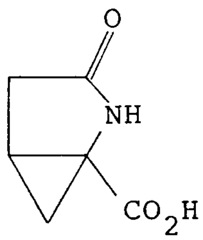 , 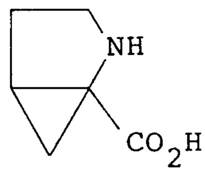 and 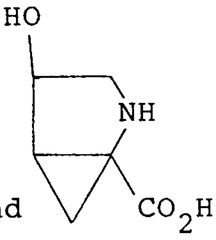

3. A compound of claim 1 wherein $R^1$ and $R^2$ are $CH_3$ and wherein the compound is 2,2 dimethyl-1-aminocyclopropane-1-carboxylic acid, a valine analog.

4. A compound of claim 1 wherein $R^1$ is $(CH_3)_2CH$ and $R^2$ is H, respectively, or wherein $R^1$ is H and $R^2$ is $(CH_3)_2CH$, respectively, and wherein the compound is 2-isopropyl-1-aminocyclopropane-1-carboxylic acid, a leucine analog.

5. A compound of claim 1, wherein $R^1$ is $CO_2H$ and $R^2$ is H, respectively, or wherein $R^1$ is H and $R^2$ is $CO_2H$, respectively, and wherein the compound is 1-aminocyclopropane-1,2-dicarboxylic acid, an aspartyl analog.

6. A compound of claim 1, wherein $R^1$ is $CH_2CO_2H$ and $R^2$ is H, respectively, or wherein $R^1$ is H and $R^2$ is $CH_2CO_2H$, respectively, and wherein the compound is 2-(2-acetyl)-1-aminocyclopropane-1-carboxylic acid, a glutamic analog.

7. A compound of claim 1 wherein $R^1$ is $CH_3SCH_2$ and $R^2$ is H, respectively, or wherein $R^1$ is H and $R^2$ is $CH_3SCH_2$, respectively, and wherein the compound is 2-methylthiomethyl-1-aminocyclopropane-1-carboxylic acid, a methionine analog.

8. A compound of claim 1 wherein $R^1$ is 3-indolyl and $R^2$ is H, respectively, or wherein $R^1$ is H and $R^2$ is 3-indolyl and wherein the compound is 2-(3-indolyl)-1-aminocyclopropane-1-carboxylic acid, a tryptophane analog.

9. A process for synthesizing a 1-amino cyclopropane-1-carboxylic acid selected from the group consisting of (1S)-E-, (1R)-E-, (1s)-Z-, (1R)-Z-, (1S)-, (1R)-, (1RS)-E-, and (1RS)-Z-isomers with respect to the carbon atom in 1-position comprising the following steps:

a) reacting a diazo compound having the formula $R^1R^2CN_2$

wherein $R^1$ and $R^2$ are, in either order, a pair of substituents selected from the group consisting of $-CH_3$ and $-CH_3$, $-CH(CH_3)_2$ and -H, $-CO_2H$ and -H, $-CH_2CO_2H$ and -H, $-CH_2SCH_3$ and -H, $-(CH_2)_3NH_2$ and -H, $-CH_2CH_2NHC(=NH)NH_2$ and -H, $-(CH_2)_2NH_2$ and -H, $-C_6H_5$ and -H, -H and -H, 4-hydroxyphenyl and -H, 3,4-dihydroxyphenyl and -H, 3-indolyl and -H, 5-hydroxy-3-indolyl and -H, -OH and -H, -SH and -H, $-CH_2SH$ and -H, $-CH_2OH$ and -H, and 4(5)-imidazolyl and -H,

with a dehydroalanine derivative having the formula

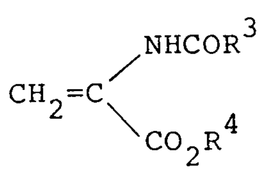

wherein $R^3$ is selected from the group consisting of an alkyl group, an aromatic group, an alkoxy group, and an aryloxy group and wherein $R^4$ is selected from the group consisting of an alkyl group and an aryl group to produce an initial reaction product;

b) decomposing the initial reaction product to produce a 1-aminocyclopropane-1-carboxylic acid derivative having the formula

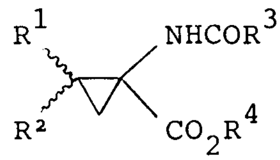

wherein the 1-amincocyclopropane-1-carboxylic acid derivative is a mixture of stereoisomers;

c) separating the mixture of stereoisomers by physical means into E- and Z-diastereomers wherein the E- and Z-diastereomers comprise a pair of enantiomers;

d) separating the pair of enantiomers by standard resolution means to produce a stereo specific 1-aminocyclopropane-1-carboxylic acid derivative;

e) deblocking the initial reaction product to produce a stereo specific 1-aminocyclopropane-1-carboxylic acid having the formula

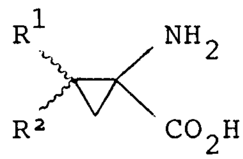

**10.** A process of claim 9 wherein the stereo specific 1-aminocyclopropane-1-carboxylic acid derivative is C-terminal deblocked to produce a stereo specific cyclopropyl acid having the formula

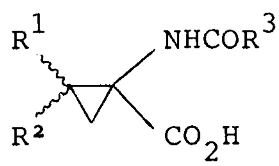

**11.** A process of claim 9 wherein N-terminal deblocking precedes C-terminal deblocking to produce the stereo specific 1-aminocyclopropane-1-carboxylic acid.

**12.** A process of claim 9 wherein $R^1$ is $(CH_3)_2CH$ and $R^2$ is H, respectively, or wherein $R^2$ is $(CH_3)_2CH$ and $R^1$ is H, respectively, to produce 2-isopropyl-1-aminocyclopropane-1-carboxylic acid, a leucine analog.

**13.** A process of claim 9 wherein $R^1$ is $CO_2H$ and $R^2$ is H, respectively, or wherein $R^2$ is $CO_2H$ and $R^1$ is H, respectively, the carboxyl group is $R^1$ or $R^2$ being protected during synthesis by standard means, to produce 1-aminocyclopropane-1,2-dicarboxylic acid, an aspartic analog.

**14.** A process of claim 9 wherein R1 is $C_6H_5$ and $R^2$ is H, respectively or wherein $R^2$ is $C_6H_5$ and $R^1$ is H, respectively, to produce 2-phenyl-1-aminocyclopropane- 1-carboxylic acid, a phenylalanine analog.

**15.** A process of claim 9 wherein $R^1$ is 4-$HOC_6H_4$ and $R^2$ is H, respectively, or wherein $R^2$ is 4-$HOC_6H_4$ and $R^1$ is H, respectively, the phenolic hydroxyl group is $R^1$ or $R^2$ being protected during synthesis by standard means, to produce 2-(4-hydroxyphenyl)-1-aminocyclopropane-1- carboxylic acid, a tyrosine analog.

**16.** A process of claim 9 wherein R1 is 3-indolyl and $R^2$ is H, respectively, or wherein $R^2$ is 3-indolyl and $R^1$ is H, respectively, to produce 2-(3-indolyl)-1-aminocyclopropane-1-carboxylic acid, a tryptophan analog.

**17.** A peptide or their esters having at least two and not more than twenty amino acid residues, wherein a peptide of known biological activity has, in lieu of at least one amino acid residue, a stereoisomer of a 1-aminocyclopropane-1-carboxylic acid selected from the group consisting of (1S)-E-, (1R)-E-, (1S)-Z-, (1R)-Z-, (1S)-, (1R)-, (1RS)-E-, and (1RS)-Z- stereoisomers of said amino acid residue with respect to

the carbon in the 1-position, said stereoisomers having the formula

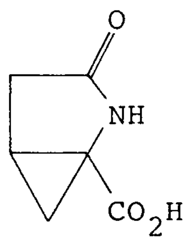

, 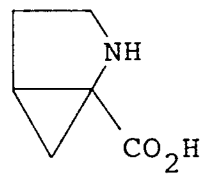

, 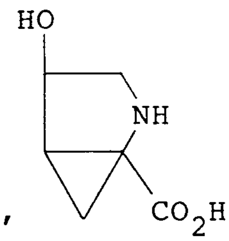

1 2 3

or 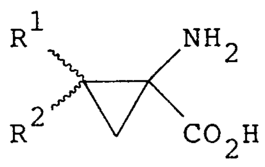

4

wherein $R^1$ and $R^2$ are, in either order, a pair of substituents selected from the group consisting of $-CH_3$ and $-CH_3$, $-CH(CH_3)_2$ and -H, $-CO_2H$ and -H, $-CH_2CO_2H$ and -H, $-CH_2SCH_3$ and -H, $-(CH_2)_3NH_2$ and -H, $-CH_2CH_2NHC(=NH)NH_2$ and -H, $-(CH_2)_2NH_2$ and -H, $-C_6H_5$ and -H, 4-hydroxyphenyl and -H, 3,4-dihydroxyphenyl and -H, 3-indolyl and -H, 5-hydroxy-3-indolyl and -H, -OH and -H, -SH and -H, $-CH_2SH$ and -H, $-CH_2OH$ and -H, and 4(5)-imidazolyl and -H.

**18.** A peptide compound according to claim 17 having the formula $R^A$-$R^B$ wherein $R^A$ is 1-aminocyclopropane-1, -2-dicarboxylic acid and $R^B$ is phenylalanine and the methyl ester thereof or wherein $R^A$ is 1-aminocyclopropane-1,2-dicarboxylic acid and $R^B$ is 2-phenyl-1-aminocyclopropane-1-carboxylic acid and the methyl ester, thereof.

**19.** A dipeptide compound according to claim 17 of the formula

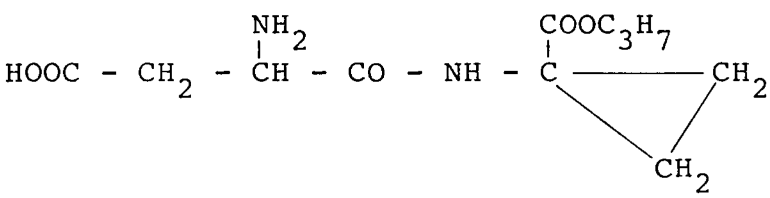

or Asp-VAla.$OC_3H_7$ wherein Asp is aspartyl acid and VAla is 1-aminocyclopropane-1-carboxylic acid.

**20.** A dipeptide compound of claim 19 wherein aspartyl is in the L form.

**21.** A peptide compound according to claim 17 having the formula $R^A$- $R^B$- $R^C$- $R^D$- $R^E$- $R^F$- $R^G$- wherein each R is an amino acid residue and wherein $R^A$ is proline, $R^B$ is phenylalanine, $R^C$ is histidine, $R^D$ is 2-isopropyl-1-aminocyclopropane- 1-carboxylic acid, $R^E$ is leucine, $R^F$ is valine, and $R^G$ is tyrosine, respectively, or wherein $R^A$ is proline, $R^B$ is phenylalanine, $R^C$ is histidine, $R^D$ is 2-isopropyl-1-aminocyclopropane- 1-carboxylic acid, $R^E$ is leucine, $R^F$ is valine, and $R^G$ is proline.

**22.** A process for synthesizing a peptide of claims 17 to 21 having at least two amino acid residues selected from the group consisting of D- or L-isomers of amino acid residues, wherein at least one amino acid residue is a stereo specific 1-aminocyclopropane-1-carboxylic acid residue comprising the following steps

a) reacting a diazo compound having the formula $R^1R^2CN_2$

wherein $R^1$ and $R^2$ are, in either order, a pair of substituents selected from the group consisting of $-CH_3$ and $-CH_3$, $-CH(CH_3)_2$ and -H, $-CO_2H$ and -H, $-CH_2CO_2H$ and -H, $-CH_2SCH_3$ and -H, $-(CH_2)_3NH_2$ and -H, $-CH_2CH_2NHC(=NH)NH_2$ and -H, $-(CH_2)_2NH_2$ and -H, $-C_6H_5$ and -H, -H and -H, 4-hydroxyphenyl and -H, 3,4-dihydroxyphenyl and -H, 3-indolyl and -H, 5-hydroxy-3-indolyl and -H, -OH and -H, -SH and -H, $-CH_2SH$ and -H, $-CH_2OH$ and -H, and 4(5)-imidazolyl and -H,

with a dehydroalanine derivative having the formula

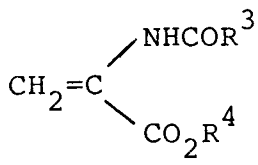

wherein $R^3$ is selected from the group consisting of an alkyl group, an aromatic group, an alkoxy group, and an aryloxy group and wherein $R^4$ is selected from the group consisting of an alkyl group and an aryl group to produce an initial reaction product;
b) decomposing the initial reaction product to produce a 1-aminocyclopropane-1-carboxylic acid derivative having the formula

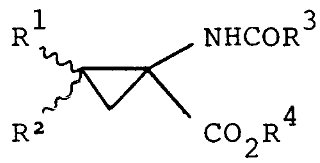

wherein the 1-aminocyclopropane-1-carboxylic acid derivative is a mixture of stereoisomers;
c) separating the mixture of stereoisomers by physical means into E- and Z-diastereomers wherein the E- and Z-diasteromers comprise a pair of enantiomers;
d) separating the pair of enantiomers by standard resolution means to produce a stereo specific 1-aminocyclopropane-1-carboxylic acid derivative;
e) deblocking the 1-aminocyclopropane-1-carboxylic acid derivative to produce an N-terminal blocked stereo specific 1-aminocyclopropane-1-carboxylic acid;
f) coupling the N-terminal blocked stereo specific 1-aminocyclopropane-1-carboxylic acid with a C-terminal blocked amino acid or peptide; and
g) repeating the above steps as necessary to produce a desired peptide.

**23.** Use of Asp-∇Ala•$OC_3H_7$ as food and beverage sweetener.

**24.** A flavored beverage composition comprising an edible acidic substance, an edible flavor, an edible color ingredient and a sweetening ingredient which consists of a dipeptide of the formula

Asp-∇Ala•$OC_3H_7$

**Claims for the following Contracting State : AT**

**1.** A process for synthesizing a 1-amino cyclopropane-1-carboxylic acid selected from the group consisting of (1S)-E-, (1R)-E-, (1s)-Z-, (1R)-Z-, (1S)-, (1R)-, (1RS)-E-, and (1RS)-Z-isomers with respect to the carbon atom in 1-position comprising the following steps:
a) reacting a diazo compound having the formula $R^1R^2CN_2$
wherein $R^1$ and $R^2$ are, in either order, a pair of substituents selected from the group consisting of $-CH_3$ and $-CH_3$, $-CH(CH_3)_2$ and -H, $-CO_2H$ and -H, $-CH_2CO_2H$ and -H, $-CH_2SCH_3$ and -H, $-(CH_2)_3NH_2$ and -H, $-CH_2CH_2NHC(=NH)NH_2$ and -H, $-(CH_2)_2NH_2$ and -H, $-C_6H_5$ and -H, -H and -H, 4-hydroxyphenyl and -H, 3,4-dihydroxyphenyl and -H, 3-indolyl and -H, 5-hydroxy-3-indolyl and -H, -OH and -H, -SH and -H, $-CH_2SH$ and -H, $-CH_2OH$ and -H, and 4(5)-imidazolyl and -H,

with a dehydroalanine derivative having the formula

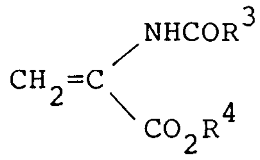

wherein $R^3$ is selected from the group consisting of an alkyl group, an aromatic group, an alkoxy group, and an aryloxy group and wherein $R^4$ is selected from the group consisting of an alkyl group and an aryl group to produce an initial reaction product;

b) decomposing the initial reaction product to produce a 1-aminocyclopropane-1-carboxylic acid derivative having the formula

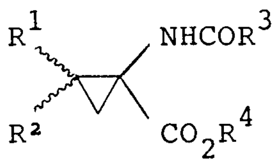

wherein the 1-aminocyclopropane-1-carboxylic acid derivative is a mixture of stereoisomers;

c) separating the mixture of stereoisomers by physical means into E- and Z-diastereomers wherein the E- and Z-diastereomers comprise a pair of enantiomers;

d) separating the pair of enantiomers by standard resolution means to produce a stereo specific 1-aminocyclopropane-1-carboxylic acid derivative;

e) deblocking the initial reaction product to produce a stereo specific 1-aminocyclopropane-1-carboxylic acid having the formula

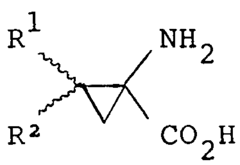

**2.** A process of claim 1 wherein the stereo specific 1-aminocyclopropane-1-carboxylic acid derivative is C-terminal deblocked to produce a stereo specific cyclopropyl acid having the formula

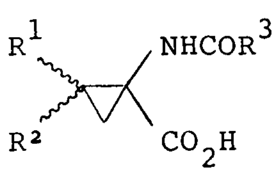

**3.** A process of claim 1 wherein N-terminal deblocking precedes C-terminal deblocking to produce the stereo specific 1-aminocyclopropane-1-carboxylic acid.

**4.** A process of claim 1 wherein $R^1$ is $(CH_3)_2CH$ and $R^2$ is H, respectively, or wherein $R^2$ is $(CH_3)_2CH$ and $R^1$ is H, respectively, to produce 2-isopropyl-1-aminocyclopropane-1-carboxylic acid, a leucine analog.

**5.** A process of claim 1 wherein $R^1$ is $CO_2H$ and $R^2$ is H, respectively, or wherein $R^2$ is $CO_2H$ and $R^1$ is H, respectively, the carboxyl group in $R^1$ or $R^2$ being protected during synthesis by standard means, to produce 1-aminocyclopropane-1,2-dicarboxylic acid, an aspartic analog.

**6.** A process of claim 1 wherein $R^1$ is $C_6H_5$ and $R^2$ is H, respectively or wherein $R^2$ is $C_6H_5$ and $R^1$ is H, respectively, to produce 2-phenyl-1-aminocyclopropane- 1-carboxylic acid, a phenylalanine analog.

**7.** A process of claim 1 wherein $R^1$ is 4-$HOC_6H_4$ and $R^2$ is H, respectively, or wherein $R^2$ is 4-$HOC_6H_4$ and $R^1$ is H, respectively, the phenolic hydroxyl group in $R^1$ or $R^2$ being protected during synthesis by standard means, to produce 2-(4-hydroxyphenyl)-1-aminocyclopropane-1-carboxylic acid, a tyrosine analog.

**8.** A process of claim 1 wherein $R^1$ is 3-indolyl and $R^2$ is H, respectively, or wherein $R^2$ is 3-indolyl and $R^1$ is H, respectively, to produce 2-(3-indolyl)-1-aminocyclopropane-1-carboxylic acid, a tryptophan analog.

**9.** A process for synthesizing a peptide having at least two amino acid residues selected from the group consisting of D- or L-isomers of amino acid residues, wherein at least one amino acid residue is a stereo specific 1-aminocyclopropane-1-carboxylic acid residue comprising the following steps

a) reacting a diazo compound having the formula $R^1R^2CN_2$

wherein $R^1$ and $R^2$ are, in either order, a pair of substituents selected from the group consisting of $-CH_3$ and $-CH_3$, $-CH(CH_3)_2$ and -H, $-CO_2H$ and -H, $-CH_2CO_2H$ and -H, $-CH_2SCH_3$ and -H, $-(CH_2)_3NH_2$ and -H, $-CH_2CH_2NHC(=NH)NH_2$ and -H, $-(CH_2)_2NH_2$ and -H, $-C_6H_5$ and -H, -H and -H, 4-hydroxyphenyl and -H, 3,4-dihydroxyphenyl and -H, 3-indolyl and -H, 5-hydroxy-3-indolyl and -H, -OH and -H, -SH and -H, $-CH_2SH$ and -H, $-CH_2OH$ and -H, and 4(5)-imidazolyl and -H,

with a dehydroalanine derivative having the formula

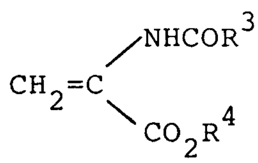

wherein $R^3$ is selected from the group consisting of an alkyl group, an aromatic group, an alkoxy group, and an aryloxy group and wherein $R^4$ is selected from the group consisting of an alkyl group and an aryl group to produce an initial reaction product;

b) decomposing the initial reaction product to produce a 1-aminocyclopropane-1-carboxylic acid derivative having the formula

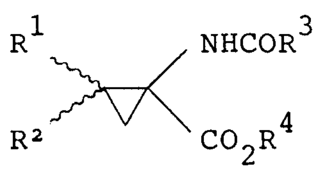

wherein the 1-aminocyclopropane-1-carboxylic acid derivative is a mixture of stereoisomers;

c) separating the mixture of stereoisomers by physical means into E- and Z-diastereomers wherein the E- and Z-diasteromers comprise a pair of enantiomers;

d) separating the pair of enantiomers by standard resolution means to produce a stereo specific 1-aminocyclopropane-1-carboxylic acid derivative;

e) deblocking the 1-aminocyclopropane-1-carboxylic acid derivative to produce an N-terminal blocked stereo specific 1-aminocyclopropane-1-carboxylic acid;

f) coupling the N-terminal blocked stereo specific 1-aminocyclopropane-1-carboxylic acid with a C-terminal blocked amino acid or peptide; and

g) repeating the above steps as necessary to produce a desired peptide.

**10.** A process according to claim 9 for synthesizing L-Aspartyl-alpha-aminocyclopropane carboxylic acid propyl ester.

**11.** Use of Asp-∇Ala•$OC_3H_7$ as food and beverage sweetener.

**Revendications**

**Revendications pour les Etat contractant suivant : BE, CH, DE, FR, GB IT, LI, LU, NL, SE**

1. Acide carbonique 1-aminocyclopropane-1 choisi parmi le groupe constitué de (1S)-E-, (1R)-E-, (1S)-Z-, (1R)-Z-, (1S)-, (1R)-, (1RS)-E- et d'isomères (1RS)-Z- qui, par rapport au carbone en position 1, ont la formule

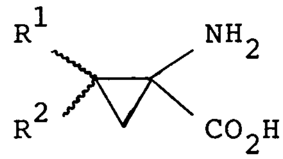

dans laquelle $R^1$ et $R^2$ représentent, dans un ordre quelconque, un couple de substituants choisi parmi le groupe de $-CH_3$ et $-CH_3$, $-CH(CH_3)_2$ et -H, $-CO_2H$ et -H, $-CH_2CO_2H$ et -H, $-CH_2SCH_3$ et -H, $-(CH_2)_3NH_2$ et -H, $-CH_2CH_2NHC-(=NH)NH_2$ et -H, $-(CH_2)_2NH_2$ et -H, 3,4-dihydroxyphényle et -H, 3-indolyle et -H, 5-hydroxy-3-indolyle et -H, -OH et -H, -SH et -H, $-CH_2SH$ et -H, $-CH_2OH$ et -H et 4(5)-imidazolyle et -H.

2. Acide carbonique cyclopropane-1 hétérocyclique choisi parmi le groupe constitué d'isomères (1S) et (1R) qui, par rapport au carbone en position 1, ont les formules

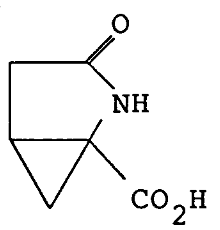 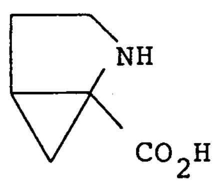 et 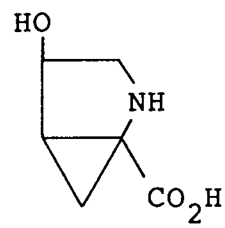

3. Composé selon la revendication 1, dans lequel $R^1$ et $R^2$ sont $CH_3$ et dans lequel le composé est un acide carbonique 2,2-diméthyle-1-aminocyclopropane-1, un analogon de valine.

4. Composé selon la revendication 1, dans lequel $R^1$ et $R^2$ sont respectivement $(CH_3)_2CH$ et H ou dans lequel $R^1$ et $R^2$ sont respectivement H et $(CH_3)_2CH$ et dans lequel le composé est un acide carbonique 2-isopropyle-1-aminocyclopropane-1, un analogon de leucine.

5. Composé selon la revendication 1, dans lequel $R^1$ et $R^2$ sont respectivement $CO_2H$ et H ou dans lequel $R^1$ et $R^2$ sont respectivement H et $CO_2H$ et dans lequel le composé est un acide dicarbonique 1-aminocyclopropane-1,2, un analogon d'aspartyle.

6. Composé selon la revendication 1, dans lequel $R^1$ et $R^2$ sont respectivement $CH_2CO_2H$ et H ou dans lequel $R^1$ et $R^2$ sont respectivement H et $CH_2CO_2H$ et dans lequel le composé est un acide carbonique 2(2-acétyle)-1-aminocyclopropane, un analogon de glutamine.

7. Composé selon la revendication 1, dans lequel $R^1$ et $R^2$ sont respectivement $CH_3SCH_2$ et H ou dans lequel $R^1$ et $R^2$ sont respectivement H et $CH_3SCH_2$ et dans lequel le composé est un acide carbonique 2-méthyle-thiométhyle-1-aminocyclopropane-1, un analogon de méthionine.

8. Composé selon la revendication 1, dans lequel $R^1$ et $R^2$ sont respectivement l'indolyle-3 et H ou dans lequel $R^1$ et $R^2$ sont respectivement H et l'indolyle-3 et dans lequel le composé est un acide carbonique 2-(3-indolyle)-1-aminocyclopropane-1, un analogon de tryptophane.

9. Procédé de synthèse d'un acide carbonique 1-aminocyclopropane-1, choisi parmi le groupe constitué d'isomères (1S)-E-, (1R)-E-, (1S)-Z-, (1R)-Z-, (1S)-, (1R)-, (1RS)-E- et (1RS)-Z- qui, par rapport à l'atome carbonique en position 1, comporte les étapes suivantes :

a) Transformation d'un composé diazoique de la formule $R^1R^2CN_2$,

dans laquelle $R^1$ et $R^2$ sont, dans un ordre quelconque, un couple de substituants choisis parmi le

groupe constitué de $-CH_3$ et $-CH_3$, $-CH(CH_3)_2$ et -H, $-CO_2H$ et -H, $-CH_2CO_2H$ et -H, $-CH_2SCH_3$ et -H, $-C(CH_2)_3NH_2$ et -H, $-CH_2CH_2NHC(=NH)NH_2$ et -H, $-(CH_2)_2NH_2$ et -H, $-C_6H_5$ et -H, -H et H 4-hydroxylphényle et -H, 3,4-dihydroxyphényle et -H, 3-indolyle et -H, 5-hydroxy-3-indolyle et -H, -OH et -H, -SH et -H,$-CH_2SH$ et -H, $-CH_2OH$ et -H et 4(5)-imidazolyle et -H,

avec un dérivé déhydro-alanine de la formule

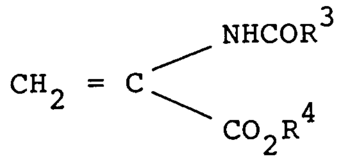

dans laquelle $R^3$ est choisi parmi le groupe composé d'un groupe alkyle, d'un groupe aromatique, d'un groupe alkoxy et d'un groupe aryloxy et dans laquelle $R^4$ est choisi parmi le groupe composé d'un groupe alkyle et d'un groupe aryle afin de fabriquer un pré-produit ;
b) Dissolution du pré-produit pour fabriquer un dérivé d'acide carbonique 1-aminocyclopropane-1 de la formule

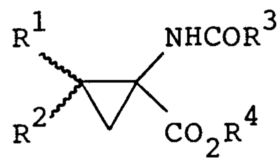

dans laquelle le dérivé d'acide carbonique 1-aminocyclopropane-1 est un mélange de stéréoisomères ;
c) Séparation du mélange de stéréo-isomères par agent physique en diastéréo-isomères E et Z dans lesquels les diastéréo-isomères E et Z comprennent un couple d'énantiomères;
d) Séparation du couple d'énantiomères par agent de séparation standard pour fabriquer un dérivé d'acide carbonique 1-aminocyclopropane-1 stéréospécifique ;
e) Déblocage du pré-produit pour fabriquer un acide carbonique 1-aminocyclopropane-1 stéréospécifique de la formule

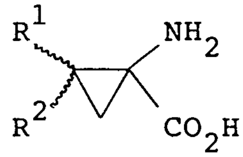

**10.** Procédé selon la revendication 9, dans lequel le dérivé d'acide carbonique 1-aminocyclopropane-1 stéréospécifique est débloqué C-terminalement afin de fabriquer un acide cyclopropyle stéréospécifique de la formule

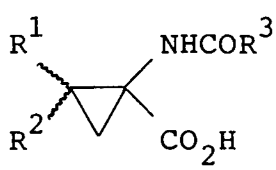

**11.** Procédé selon la revendication 9, dans lequel le déblocage N-terminal précède un déblocage C-terminal pour fabriquer l'acide carbonique 1-aminocyclopropane-1 stéréospécifique.

**12.** Procédé selon la revendication 9, dans lequel $R^1$ et $R^2$ sont respectivement $(CH_3)_2CH$ et H ou dans

lequel $R^2$ et $R^1$ sont respectivement $(CH_3)_2CH$ et H pour fabriquer un acide carbonique 2-isopropyle-1-aminocyclopropane-1, un analogon de leucine.

**13.** Procédé selon la revendication 9, dans lequel $R^1$ et $R^2$ sont respectivement $CO_2H$ et H ou dans lequel $R^2$ et $R^1$ sont respectivement $CO_2H$ et H, le groupe carboxyle est protégé dans $R^1$ ou $R^2$ pendant la synthèse par moyen usuel pour fabriquer un acide dicarbonique 1-aminocyclopropane-1,2, un analogon d'asparagine.

**14.** Procédé selon la revendication 9, dans lequel $R^1$ et $R^2$ sont respectivement $C_6H_5$ et H ou dans lequel $R^2$ et $R^1$ sont respectivement $C_6H_5$ et H pour fabriquer un acide carbonique 2-phényle-1-aminocyclopropane-1, un analogon de phényle-alanine.

**15.** Procédé selon la revendication 9, dans lequel $R^1$ et $R^2$ sont respectivement $4\text{-}HOC_6H_4$ et H ou dans lequel $R^2$ et $R^1$ sont respectivement $4\text{-}HOC_6H_4$ et H, le groupe hydroxyle phénolique est protégé dans $R^1$ ou $R^2$ pendant la synthèse par moyen usuel pour fabriquer un acide carbonique 2-(4-hydroxyphényle)-1-aminocyclopropane-1, un analogon de tyrosine.

**16.** Procédé selon la revendication 9, dans lequel $R^1$ et $R^2$ sont respectivement l'indolyle-3 et H ou dans lequel $R^2$ et $R^1$ sont respectivement l'indolyle-3 et H pour fabriquer un acide carbonique 2-(3-indolyle)-1-aminocyclopropane-1, un analogon de tryptophane.

**17.** Peptide ou ses esters ayant au moins deux et pas plus de vingt radicaux amino-acides, dans lequel le peptide d'activité biologique connue a, au lieu d'au moins un radical amino-acide un stéréo-isomère d'un acide 1-aminocyclopropane-1, choisi parmi le groupe constitué de stéréo-isomères (1S)-E-, (1R)-E-, (1S)-Z-, (1R)-Z-, (1S)-, (1R)-, (1RS)-E- et 1RS-Z de ce radical amino-acide et ces stéréo-isomères, par rapport au carbone en position 1, ont la formule

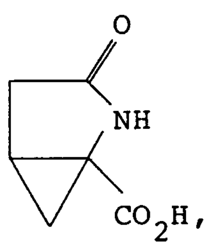

1

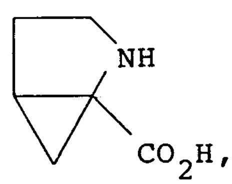

2

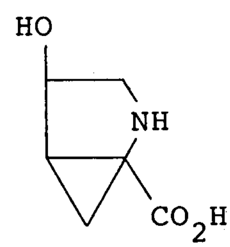

3

ou

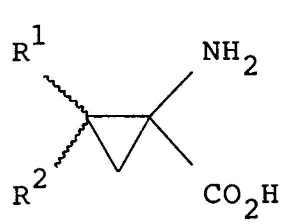

4

dans laquelle $R^1$ et $R^2$ sont, dans un ordre quelconque, un couple de substituants choisi parmi le groupe constitué de $-CH_3$ et $-CH_3$, $-CH(CH_3)_2$ et -H, $-CO_2H$ et -H, $-CH_2CO_2H$ et -H, $-CH_2SCH_3$ et -H, $-(CH_2)_3NH_2$, et -H, $-CH_2CH_2NHC(=NH)NH_2$ et -H, $-(CH_2)_2NH_2$ et -H, $-C_6H_5$ et -H, 4-hydroxyphényle et -H, 3,4-dihydroxyphényle et -H, 3-indolyle et -H, 5-hydroxy-3-indolyle et -H, -OH et -H, -SH et -H, $-CH_2SH$ et -H, $-CH_2OH$ et -H, et 4(5)-imidazolyle et -H.

**18.** Composé de peptide selon la revendication 17 de la formule $R^A\text{-}R^B$, dans lequel $R^A$ est un acide

dicarbonique 1-aminocyclopropane-1-2 et $R^B$ une phénylealanine et son ester de méthyle ou dans lequel $R^A$ est un acide dicarbonique 1-aminocyclopropane-1,2 et $R^B$ un acide carbonique 2-phényle-1-aminocyclopropane-1 et son ester de méthyle.

**19.** Composé dipeptide selon la revendication 17 de la formule

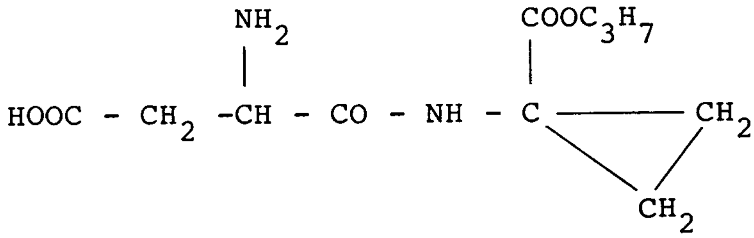

ou Asp-∇Ala . $OC_3H_7$, dans laquelle Asp est l'aspartyle et ∇Ala un acide carbonique 1-aminocyclopropane-1.

**20.** Composé dipeptide selon la revendicaiton 19, dans laquelle l'aspartyle se présente sous la forme L.

**21.** Composé peptide selon la revendication 17 de la formule $R^A$ - $R^B$ - $R^C$ - $R^D$ - $R^E$ - $R^F$ - $R^G$-, dans laquelle chaque R est un radical amino-acide et dans laquelle $R^A$, $R^B$, $R^C$, $R^D$, $R^E$, $R^F$ et $R^G$ sont respectivement la proline, la phényle-alanine, l'histidine, un acide carbonique 2-isopropyle-1-aminocyclopropane-1, la leucine, la valine et la tyrosine ou dans laquelle $R^A$ est la proline, $R^B$ la phényle-alanine, $R^C$ l'histidine, $R^D$ un acide carbonique 2-isopropyle-1-aminocyclopropane-1, $R^E$ la leucine, $R^F$ la valine et $R^G$ la proline.

**22.** Procédé de synthèse d'un peptide selon l'une des revendications 17 à 21 ayant au moins deux radicaux amino-acides choisis parmi le groupe constitué d'isomères, D ou L de radicaux amino-acides, dans lequel au moins un radical amino-acide est un radical d'acide carbonique 1-aminocyclopropane-1 stéréospécifique comprenant les étapes suivantes :

a) Transformation d'un composé diazoique de la formule $R^1R^2CN^2$,

dans laquelle $R^1$ et $R^2$ sont, dans un ordre quelconque, un couple de substituants choisi parmi le groupe constitué de $-CH_3$ et $-CH_3$, $-CH(CH_3)_2$ et -H, $-CO_2H$ et -H, $-CH_2CO_2H$ et -H, $-CH_2SCH_3$ et -H, $-(CH_2)_3NH_2$ et -H, $-CH_2CH_2NHC(=NH)NH_2$ et -H, $(CH_2)_2NH_2$ et -H, $-C_6H_5$ et -H, -H et -H 4-hydroxyphényle et -H 3,4-dihydroxyphényle et -H, 3-indolyle et -H, 5-hydroxy-3- indolyle et -H, -OH et -H, -SH et -H, $-CH_2SH$ et -H, $CH_2OH$ et -H et 4(5)-imidazolyle et -H,

avec un dérivé de déhydro-alanine de la formule

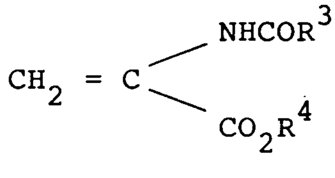

dans laquelle $R^3$ est choisi parmi le groupe composé d'un groupe alkyle, d'un groupe aromatique, d'un groupe alkoxy et d'un groupe aryloxy et dans laquelle $R^4$ est choisi parmi le groupe composé d'un groupe alkyle et d'un groupe aryl pour fabriquer un pré-produit ;

b) Dissolution du pré-produit pour fabriquer un dérivé d'acide carbonique 1-aminocyclopropane-1 de la formule

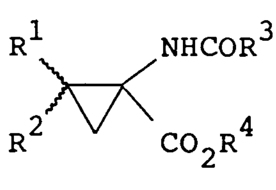

dans laquelle le dérivé d'acide carbonique 1-aminocyclopropane-1 est un mélange de stéréoisomères ;
c) Séparation du mélange de stéréo-isomères par agent physique et diastomères E et Z, dans lequel les diastomères E et Z comprennent un couple d'énantiomères ;
d) Séparation du couple d'énantiomères par agent de séparation standard pour fabriquer un dérivé d'acide carbonique 1-aminocyclopropane-1 stéréospécifique ;
e) Déblocage du dérivé d'acide carbonique 1-aminocyclopropane-1 pour fabriquer un acide carbonique 1-aminocyclopropane-1 stéréospécifique bloqué N-terminalement ;
f) Liaison de l'acide carbonique 1-aminocyclopropane-1 stéréospécifique bloqué N-terminalement avec un amino-acide bloqué C-terminalement ou avec un peptide ; et
g) Répétition des étapes sus-nommées telles que nécessaires pour la fabrication du peptide souhaité.

**23.** Utilisation d'Asp-∇Ala . $OC_3H_7$ comme saccharine pour produits alimentaires et boissons.

**24.** Boisson composée aromatisée comprenant une substance d'acidification comestible, un arôme comestible, un colorant comestible et une saccharine constituée d'un dipeptide de la formule Asp-∇Ala . $OC_3H_7$.

**Revendications pour l'Etat contractants suivant : AT**

**1.** Procédé de synthèse d'un acide carbonique 1-aminocyclopropane-1 choisi parmi le groupe constitué d'isomères (1S)-E-, (1R)-E-, (1S)-Z-, (1R)-Z-, (1S)-, (1R)-, (1RS)-E- et (1RS)-Z- qui, par rapport à l'atome carbonique en position 1, comporte les étapes suivantes :
a) Transformation d'un composé diazoique de la formule $R^1R^2CN_2$
dans laquelle $R^1$ et $R^2$, dans un ordre quelconque, sont un couple de substituants choisi parmi le groupe composé de $-CH_3$ et $-CH_3$, $-CH(CH_3)_2$ et -H, $-CO_2H$ et -H, $-CH_2CO_2H$ et -H, $-CH_2SCH_3$ et -H, $-(CH_2)_3NH_2$ et -H, $-CH_2CH_2NHC(=NH)NH_2$ et -H, $-(CH_2)_2NH_2$ et -H, $-C_6H_5$ et -H, -H et -H, 4-hydroxyphényle et -H, 3,4-dihydroxyphényle et -H, 3-indolyle et -H, 5-hydroxy-3-indolyle et -H, -OH et -H, -SH et -H, $-CH_2SH$ et -H, $-CH_2OH$ et -H et 4(5)-imidazolyle et -H,

avec un dérivé de déhydro-alanine de la formule

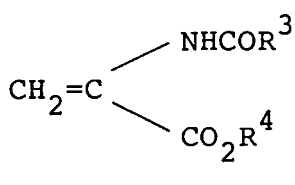

dans laquelle $R^3$ est choisi parmi le groupe constitué d'un groupe alkyle, d'un groupe aromatique, d'un groupe alkoxy et d'un groupe aryloxy et dans laquelle $R^4$ est choisi parmi le groupe composé d'un groupe alkyle et d'un groupe aryle pour fabriquer un pré-produit ;
b) Dissolution du pré-produit pour fabriquer un dérivé d'acide carbonique 1-aminocyclopropane-1 de la formule

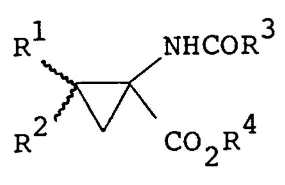

dans laquelle le dérivé d'acide carbonique 1-aminocyclopropane-1 est un mélange de stéréo-isomères ; c) Séparation du mélange de stéréo-isomères par agent physique en diastéréomères E et Z, dans lequel les diastéréomères E et Z comprennent un couple d'énantiomères ;
d) Séparation du mélange d'énantiomères par agent de séparation usuel pour fabriquer un dérivé d'acide carbonique 1-aminocyclopropane-1 stéréospécifique ;

e) Déblocage du pré-produit pour la fabrication d'un acide carbonique 1-aminocyclopropane-1 stéréospécifique de la formule

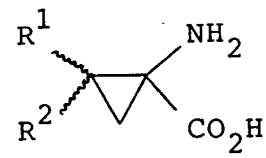

2. Procédé selon la revendication 1, dans lequel le dérivé d'acide carbonique 1-aminocyclopropane-1 stéréospécifique est débloqué C-terminalement pour fabriquer un acide cyclopropyle stéréospécifique de la formule

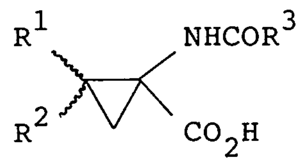

3. Procédé selon la revendication 1, dans lequel le déblocage N-terminal précède un déblocage C-terminal pour fabriquer l'acide carbonique 1-aminocyclopropane-1 stéréospécifique.

4. Procédé selon la revendication 1, dans lequel $R^1$ et $R^2$ sont respectivement $(CH_3)_2CH$ et H ou dans lequel $R^2$ et $R^1$ sont respectivement $(CH_3)_2CH$ et H pour fabriquer un acide carbonique 2-isopropyle-1-aminocyclopropane-1, un analogon de leucine.

5. Procédé selon la revendication 1, dans lequel $R^1$ et $R^2$ sont respectivement $CO_2H$ et H ou dans lequel $R^2$ et $R^1$ sont respectivement $CO_2H$ et H, le groupe carboxyle est protégé dans $R^1$ ou $R^2$ pendant la synthèse par moyen usuel pour fabriquer un acide dicarbonique 1-aminocyclopropane-1,2, un analogon d'asparagine.

6. Procédé selon la revendication 1, dans lequel $R^1$ et $R^2$ sont respectivement $C_6H_5$ et H ou dans lequel $R^2$ et $R^1$ sont respectivement $C_6H_5$ et H pour fabriquer un acide carbonique 2-phényle-1-aminocyclopropane-1, un analogon de phényle-alanine.

7. Procédé selon la revendication 1, dans lequel $R^1$ et $R^2$ sont respectivement $4\text{-}HOC_6H_4$ et H ou dans lequel $R^2$ et $R^1$ sont respectivement $4\text{-}HOC_6H_4$ et H, le groupe phénol-hydroxyle est protégé dans $R^1$ ou $R^2$ pendant la synthèse par un moyen usuel pour fabriquer un acide carbonique 2-(4-hydroxyphényle) -1-aminocyclopropane-1, un analogon de tyrosine.

8. Procédé selon la revendication 1, dans lequel $R^1$ et $R^2$ sont respectivement l'indolyle-3 et H ou dans lequel $R^2$ et $R^1$ sont respectivement l'indolyle-3 et H pour fabriquer un acide carbonique 2-(3-indolyle)-1-aminocyclopropane-1, un analogon de tryptophane.

9. Procédé de synthèse d'un peptide avec au moins deux radicaux amino-acides, choisi parmi le groupe constitué des isomères D ou L de radicaux amino-acides, dans lequel au moins un radical amino-acide est un radical d'acide carbonique 1-aminocyclopropane-1 stéréospécifique comprenant les étapes suivantes :

a) Transformation d'un composé diazoïque de la formule $R^1R^2CN_2$, dans laquelle $R^1$ et $R^2$ sont, dans un ordre quelconque, un couple de substituants choisi parmi le groupe constitué de $-CH_3$ et $-CH_3$, $-CH(CH_3)_2$ et -H, $-CO_2H$ et -H, $-CH_2CO_2H$ et -H, $-CH_2SCH_3$ et -H, $-(CH_2)_3NH_2$ et -H, $-CH_2CH_2NHC(=NH)NH_2$ et -H, $-(CH_2)_2NH_2$ et -H, $-C_6H_5$ et -H, -H et -H, 4-hydroxyphényle et -H, 3,4-dihydroxyphényle et -H, 3-indolyle et -H, 5-hydroxy-3-indolyle et -H, -OH et -H, -SH et -H, $-CH_2SH$ et -H, $-CH_2OH$ et -H et 4(5)-imidazolyle et -H, avec un dérivé de déhydro-analine de la formule

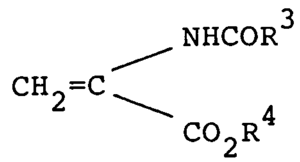

dans laquelle $R^3$ est choisi parmi le groupe composé d'un groupe alkyle, d'un groupe aromatique, d'un groupe alkoxy et d'un groupe aryloxy et dans laquelle $R^4$ est choisi parmi le groupe constitué d'un groupe alkyle et d'un groupe aryle, pour fabriquer un pré-produit ;

b) Dissolution du pré-produit pour fabriquer un dérivé d'acide carbonique 1-aminocyclopropane-1 de la formule

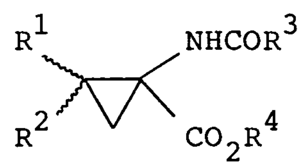

dans lequel le dérivé d'acide carbonique 1-aminocyclopropane-1 est un mélange de stéréo-isomères ;

c) Séparation du mélange de stéréo-isomères par agent physique en diastéréomères E et Z, dans lequel les diastéréomères E et Z comprennent un couple d'énantiomères ;

d) Séparation du couple d'énantiomères par agent de séparation usuel pour fabriquer un dérivé d'acide carbonique 1-aminocyclopropane-1 stéréospécifique ;

e) Déblocage du dérivé d'acide carbonique 1-aminocyclopropane-1 pour fabriquer un acide carbonique 1-aminocyclopropane-1 stéréospécifique bloqué N-terminalement ;

f) Liaison de l'acide carbonique 1-aminocyclopropane-1 stéréospécifique bloqué N-terminalement avec un amino-acide bloqué C-terminalement ou avec un peptide ; et

g) Répétition des étapes sus-nommées, telles que nécessaires pour la fabrication du peptide souhaité.

**10.** Procédé selon la revendication 9 pour la synthèse de l'ester propyle d'acide carbonique L-aspartylealpha-aminocyclopropane.

**11.** Utilisation de Asp-VAla . $OC_3H_7$ comme saccharine pour produits alimentaires et boissons.

**Patentansprüche**
**Patentansprüche für folgende Vertragsstaaten : BE CH DE FR GB IT LI LU NL SE**

**1.** Eine 1-Aminocyclopropan-1-carbonsäure, ausgewählt aus der Gruppe, bestehend aus (1S)-E-, (1R)-E-, (1S)-Z-, (1R)-Z-, (1S)-, (1R)-, (1RS)-E- und (1RS)-Z-Isomeren, die bezogen auf den Kohlenstoff in Position 1, die Formel

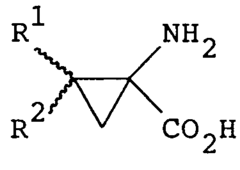

haben, in der $R^1$ und $R^2$, in beliebiger Reihenfolge, ein ausgewähltes aus der Gruppe von $-CH_3$ und $-CH_3$, $-CH(CH_3)_2$ und -H, $-CO_2H$ und -H, $-CH_2CO_2H$ und -H, $-CH_2SCH_3$ und -H, $-(CH_2)_3NH_2$ und -H, $-CH_2CH_2NHC\text{-}(=NH)NH_2$ und -H, $-(CH_2)_2NH_2$ und -H, 3,4-Dihydroxyphenyl und -H, 3-Indolyl und -H, 5-Hydroxy-3-indolyl und -H, -OH und -H, -SH und -H, $-CH_2SH$ und -H, $-CH_2OH$ und -H und 4(5)-Imidazolyl und -H ausgewähltes Substituentenpaar sind.

2. Eine heterocyclische Cyclopropan-1-carbonsäure, ausgewählt aus der Gruppe, bestehend aus (1S)- und (1R)-Isomeren, die bezogen auf den Kohlenstoff in der Position 1 die Formeln

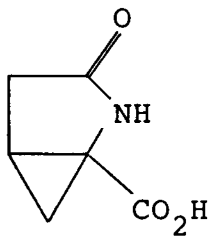
,
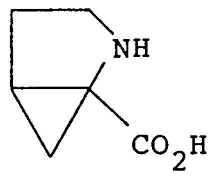
und
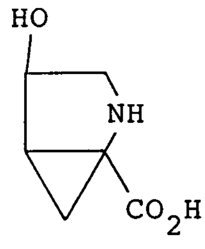

haben.

3. Eine Verbindung nach Anspruch 1, worin $R^1$ und $R^2$ $CH_3$ sind und worin die Verbindung eine 2,2-Dimethyl-1-aminocyclopropan-1-carbonsäure, ein Valinanalogon, ist.

4. Eine Verbindung nach Anspruch 1, worin jeweils $R^1$ $(CH_3)_2CH$ und $R^2$ H ist oder worin jeweils $R^1$ H ist und $R^2$ $(CH_3)_2CH$ und worin die Verbindung eine 2-Isopropyl-1-aminocyclopropan-1-carbonsäure, ein Leucinanalogon, ist.

5. Eine Verbindung nach Anspruch 1, worin jeweils $R^1$ $CO_2H$ und $R^2$ H ist oder worin jeweils $R^1$ H und $R^2$ $CO_2H$ ist und worin die Verbindung eine 1-Aminocyclopropan-1,2-dicarbonsäure, ein Aspartylanalogon, ist.

6. Eine Verbindung nach Anspruch 1, worin jeweils $R^1$ $CH_2CO_2H$ und $R^2$ H ist oder worin jeweils $R^1$ H und $R^2$ $CH_2CO_2H$ ist und worin die Verbindung eine 2-(2-Acetyl)-1-aminocyclopropan-1-carbonsäure, ein Glutaminanalogon, ist.

7. Eine Verbindung nach Anspruch 1, worin jeweils $R^1$ $CH_3SCH_2$ und $R^2$ H ist oder worin jeweils $R^1$ H und $R^2$ $CH_3SCH_2$ ist und worin die Verbindung eine 2-Methylthiomethyl-1-aminocyclopropan-1-carbonsäure, ein Methioninanalogon, ist.

8. Eine Verbindung nach Anspruch 1, worin jeweils $R^1$ 3-Indolyl und $R^2$ H ist oder worin $R^1$ H und $R^2$ 3-Indolyl ist und worin die Verbindung eine 2-(3-Indolyl)-1-aminocyclopropan-1-carbonsäure, ein Tryptophananalogon, ist.

9. Ein Verfahren zur Synthese einer 1-Aminocyclopropan-1-carbonsäure, ausgewählt aus der Gruppe, bestehend aus (1S)-E-, (1R)-E-, (1S)-Z-, (1R)-Z-, (1S)-, (1R)-, (1RS)-E- und (1RS)-Z-Isomeren, das bezogen auf das Kohlenstoffatom in der Position 1 folgende Schritte umfaßt:

a) Umsetzung einer Diazoverbindung mit der Formel $R^1R^2CN_2$
worin $R^1$ und $R^2$ in beliebiger Reihenfolge ein Substituentenpaar sind, ausgewählt aus der Gruppe, bestehend aus $-CH_3$ und $-CH_3$, $-CH(CH_3)_2$ und -H, $-CO_2H$ und -H, $-CH_2CO_2H$ und -H, $-CH_2SCH_3$ und -H, $-(CH_2)_3NH_2$ und -H, $-CH_2CH_2NHC(=NH)NH_2$ und -H, $-(CH_2)_2NH_2$ und -H, $-C_6H_5$ und -H, -H und -H, 4-Hydroxyphenyl und -H, 3,4-Dihydroxyphenyl und -H, 3-Indolyl und -H, 5-Hydroxy-3-indolyl und -H, -OH und -H, -SH und -H, $-CH_2SH$ und -H, $-CH_2OH$ und -H und 4(5)-Imidazolyl und -H,

mit einem Dehydroalaninderivat der Formel

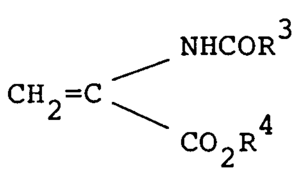

worin $R^3$ aus der aus einer Alkylgruppe, einer aromatischen Gruppe, einer Alkoxygruppe und einer

Aryloxygruppe bestehenden Gruppe ausgewählt ist und worin $R^4$ aus der aus einer Alkylgruppe und einer Arylgruppe bestehenden Gruppe ausgewählt ist, um ein Vorprodukt herzustellen;

b) Abbau des Vorprodukts, um ein 1-Aminocyclopropan-1-carbonsäurederivat mit der Formel herzustellen

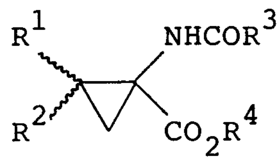

worin das 1-Aminocyclopropan-1-carbonsäurederivat ein Gemisch von Stereoisomeren ist;

c) Trennung des Stereoisomerengemischs durch physikalische Mittel in E- und Z-Diastereomere, worin die E- und Z-Diastereomere ein Enantiomerenpaar umfassen;

d) Trennung des Enantiomerenpaares durch Standardtrennmittel, um ein stereospezifisches 1-Aminocyclopropan-1-carbonsäurederivat herzustellen;

e) Deblockierung des Vorprodukts zur Herstellung einer stereospezifischen 1-Aminocyclopropan-1-carbonsäure mit der Formel

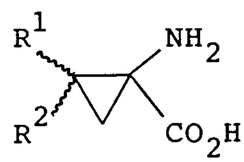

**10.** Ein Verfahren nach Anspruch 9, worin das stereospezifische 1-Aminocyclopropan-1-carbonsäurederivat C-terminal deblockiert wird, um eine stereospezifische Cyclopropylsäure der Formel

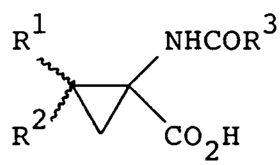

herzustellen.

**11.** Ein Verfahren nach Anspruch 9, worin die N-terminale Deblockierung einer C-terminalen Deblockierung vorausgeht, um die stereospezifische 1-Aminocyclopropan-1-carbonsäure herzustellen.

**12.** Ein Verfahren nach Anspruch 9, worin $R^1$ jeweils $(CH_3)_2CH$ und $R^2$ H ist oder worin jeweils $R^2$ $(CH_3)_2CH$ und R H ist, um eine 2-Isopropyl-1-aminocyclopropan-1-carbonsäure, ein Leucinanalogon, herzustellen.

**13.** Ein Verfahren nach Anspruch 9, worin jeweils $R^1$ $CO_2H$ und $R^2$ H ist oder worin jeweils $R^2$ $CO_2H$ und $R^1$ H ist, die Carboxylgruppe in $R^1$ oder $R^2$ während der Synthese durch übliche Mittel geschützt ist, um eine 1-Aminocyclopropan-1,2-dicarbonsäure, ein Asparaginanalogon, herzustellen.

**14.** Ein Verfahren nach Anspruch 9, worin jeweils $R^1$ $C_6H_5$ und $R^2$ H ist oder worin jeweils $R^2$ $C_6H_5$ und $R^1$ H ist, um eine 2-Phenyl-1-aminocyclopropan- 1-carbonsäure, ein Phenylalaninanalogon, herzustellen.

**15.** Ein Verfahren nach Anspruch 9, worin jeweils $R^1$ 4-$HOC_6H_4$ und $R^2$ H ist oder worin jeweils $R^2$ 4-$HOC_6H_4$ und $R^1$ H ist, die phenolische Hydroxylgruppe in $R^1$ oder $R^2$ während der Synthese durch übliche Mittel geschützt ist, um eine 2-(4-Hydroxyphenyl)-1-aminocyclopropan-1-carbonsäure, ein Tyrosinanalogon, herzustellen.

**16.** Ein Verfahren nach Anspruch 9, worin jeweils $R^1$ 3-Indolyl und $R^2$ H ist oder worin jeweils $R^2$ 3-Indolyl und $R^1$ H ist, um eine 2-(3-Indolyl)-1-aminocyclopropan-1-carbonsäure, ein Tryptophananalogon, herzustellen.

**17.** Ein Peptid oder seine Ester mit mindestens zwei und nicht mehr als zwanzig Aminosäureresten, worin das Peptid von bekannter biologischer Aktivität anstelle von wenigstens einem Aminosäurerest ein Stereoisomer einer 1-Aminocyclopropan-1-carbonsäure, ausgewählt aus der Gruppe, bestehend aus (1S)-E-, (1R)-E-, (1S)-Z-, (1R)-Z-, (1S)-, (1R)-, (1RS)-E- und (1RS)-Z-Stereoisomeren dieses Aminosäurerests hat und, diese Stereoisomere bezogen auf den Kohlenstoff in Position 1, die Formel

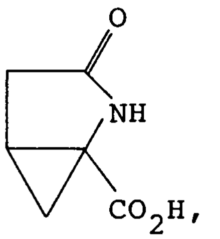
1

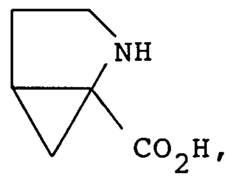
2

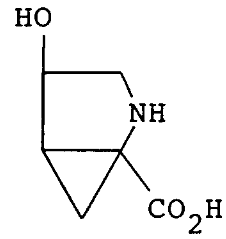
3

oder

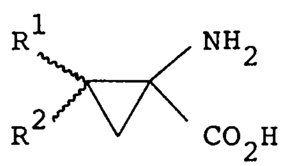
4

haben, worin $R^1$ und $R^2$ in beliebiger Reihenfolge ein Substituentenpaar sind, ausgewählt aus der Gruppe, bestehend aus $-CH_3$ und $-CH_3$, $-CH(CH_3)_2$ und -H, $-CO_2H$ und -H, $-CH_2CO_2H$ und -H, $-CH_2SCH_3$ und -H, $-(CH_2)_3NH_2$ und -H, $-CH_2CH_2NHC(=NH)NH_2$ und -H, $-(CH_2)_2NH_2$ und -H, $-C_6H_5$ und -H, 4-Hydroxyphenyl und -H, 3,4-Dihydroxyphenyl und -H, 3-Indolyl und -H, 5-Hydroxy-3-indolyl und -H, -OH und -H, -SH und -H, $-CH_2SH$ und -H, $-CH_2OH$ und -H und 4(5)-Imidazolyl und -H.

**18.** Eine Peptidverbindung nach Anspruch 17 mit der Formel $R^A$-$R^B$, worin $R^A$ eine 1-Aminocyclopropan-1-2-dicarbonsäure und $R^B$ Phenylalanin und sein Methylester ist oder worin $R^A$ eine 1-Aminocyclopropan-1,2-dicarbonsäure und $R^B$ eine 2-Phenyl-1-aminocyclopropan-1-carbonsäure und sein Methylester ist.

**19.** Eine Dipeptidverbindung nach Anspruch 17 der Formel

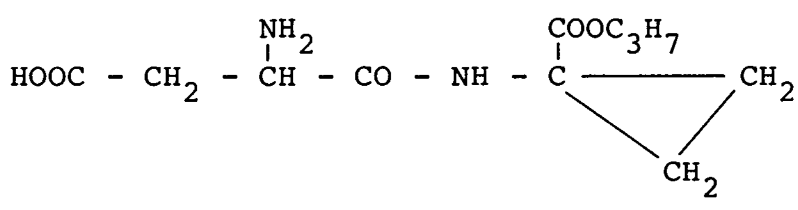

oder Asp-∇Ala.$OC_3H_7$, worin Asp Aspartyl und VAla eine 1-Aminocyclopropan-1-carbonsäure ist.

**20.** Eine Dipeptidverbindung nach Anspruch 19, worin das Aspartyl in der L-Form vorliegt.

**21.** Eine Peptidverbindung nach Anspruch 17 der Formel $R^A$-$R^B$- $R^C$- $R^D$- $R^E$- $R^F$- $R^G$-, worin jedes R ein Aminosäurerest ist und worin jeweils $R^A$ Prolin, $R^B$ Phenylalanin, $R^C$ Histidin, $R^D$ eine 2-Isopropyl-1-aminocyclopropan-1-carbonsäure, $R^E$ Leucin, $R^F$ Valin und $R^G$ Tyrosin ist oder worin $R^A$ Prolin, $R^B$ Phenylalanin, $R^C$ Histidin, $R^D$ eine 2-Isopropyl-1-aminocyclopropan-1-carbonsäure, $R^E$ Leucin, $R^F$ Valin

und $R^G$ Prolin ist.

**22.** Ein Verfahren zur Synthese eines Peptids nach einem der Ansprüche 17 bis 21 mit wenigstens zwei Aminosäureresten, ausgewählt aus der aus D- oder L-Isomeren von Aminosäureresten bestehenden Gruppe, worin wenigstens ein Aminosäurerest ein stereospezifischer 1-Aminocyclopropan-1-carbonsäurerest ist, das folgende Schritte umfaßt:

a) Umsetzung einer Diazoverbindung mit der Formel $R^1R^2CN_2$,
worin $R^1$ und $R^2$ in beliebiger Reihenfolge ein Substituentenpaar sind, ausgewählt aus der Gruppe, bestehend aus $-CH_3$ und $-CH_3$, $-CH(CH_3)_2$ und -H, $-CO_2H$ und -H, $-CH_2CO_2H$ und -H, $-CH_2SCH_3$ und -H, $-(CH_2)_3NH_2$ und -H, $-CH_2CH_2NHC(=NH)NH_2$ und -H, $-(CH_2)_2NH_2$ und -H, $-C_6H_5$ und -H, -H und -H, 4-Hydroxyphenyl und -H, 3,4-Dihydroxyphenyl und -H, 3-Indolyl und -H, 5-Hydroxy-3-indolyl und -H, -OH und -H, -SH und -H, $-CH_2SH$ und -H, $-CH_2OH$ und -H und 4(5)-Imidazolyl und -H,
mit einem Dehydroalaninderivat der Formel

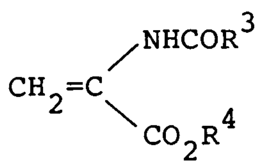

worin $R^3$ aus der aus einer Alkylgruppe, einer aromatischen Gruppe, einer Alkoxygruppe und einer Aryloxygruppe bestehenden Gruppe ausgewählt ist und worin $R^4$ aus der aus einer Alkylgruppe und einer Arylgruppe bestehenden Gruppe ausgewählt ist, um ein Vorprodukt herzustellen;

b) Abbau des Vorprodukts, um ein 1-Aminocyclopropan-1-carbonsäurederivat der Formel

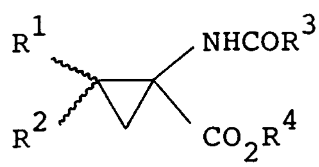

herzustellen, worin das 1-Aminocyclopropan-1-carbonsäurederivat ein Gemisch von Stereoisomeren ist;

c) Trennung des Stereoisomerengemischs durch physikalische Mittel in E- und Z-Diastereomere, worin die E- und Z-Diastereomere ein Enantiomerenpaar umfassen;

d) Trennung des Enantiomerenpaares durch Standardtrennmittel, um ein stereospezifisches 1-Aminocyclopropan-1-carbonsäurederivat herzustellen;

e) Deblockierung des 1-Aminocyclopropan-1-carbonsäure-derivats, um eine N-terminal blockierte stereospezifische 1-Aminocyclopropan-1-carbonsäure herzustellen;

f) Verknüpfung der N-terminal blockierten stereospezifischen 1-Aminocyclopropan-1-carbonsäure mit einer C-terminal blockierten Aminosäure oder einem Peptid; und

g) Wiederholung der oben genannten Verfahrensschritte, wie sie zur Herstellung des gewünschten Peptids notwendig sind.

**23.** Verwendung von Asp-∇Ala • $OC_3H_7$ als Nahrungsmittel- und Getränkesüßstoff.

**24.** Eine aromatisierte Getränkezusammensetzung, die eine genießbare Säuerungssubstanz, ein genießbares Aroma, einen genießbaren Farbstoff und einen Süßstoff, der aus einem Dipeptid der Formel Asp-∇Ala • $OC_3H_7$ besteht, umfaßt.

**Patentansprüche für folgenden Vertragsstaat : AT**

**1.** Ein Verfahren zur Synthese einer 1-Aminocyclopropan-1-carbonsäure, ausgewählt aus der Gruppe, bestehend aus (1S)-E-, (1R)-E-, (1S)-Z-, (1R)-Z-, (1S)-, (1R)-, (1RS)-E- und (1RS)-Z-Isomeren, das bezogen auf das Kohlenstoffatom in der Position 1 folgende Schritte umfaßt:

a) Umsetzung einer Diazoverbindung mit der Formel $R^1R^2CN_2$

worin $R^1$ und $R^2$ in beliebiger Reihenfolge ein Substituentenpaar sind, ausgewählt aus der Gruppe, bestehend aus $-CH_3$ und $-CH_3$, $-CH(CH_3)_2$ und -H, $-CO_2H$ und -H, $-CH_2CO_2H$ und -H, $-CH_2SCH_3$ und -H, $-(CH_2)_3NH_2$ und -H, $-CH_2CH_2NHC(=NH)NH_2$ und -H, $-(CH_2)_2NH_2$ und -H, $-C_6H_5$ und -H, -H und -H, 4-Hydroxyphenyl und -H, 3,4-Dihydroxyphenyl und -H, 3-Indolyl und -H, 5-Hydroxy-3-indolyl und -H, -OH und -H, -SH und -H, $-CH_2SH$ und -H, $-CH_2OH$ und -H und 4(5)-Imidazolyl und -H,

mit einem Dehydroalaninderivat der Formel

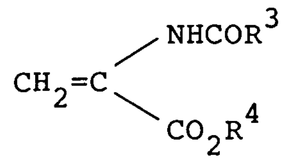

worin $R^3$ aus der aus einer Alkylgruppe, einer aromatischen Gruppe, einer Alkoxygruppe und einer Aryloxygruppe bestehenden Gruppe ausgewählt ist und worin $R^4$ aus der aus einer Alkylgruppe und einer Arylgruppe bestehenden Gruppe ausgewählt ist, um ein Vorprodukt herzustellen;

b) Abbau des Vorprodukts, um ein 1-Aminocyclopropan-1-carbonsäurederivat mit der Formel herzustellen

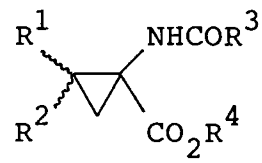

worin das 1-Aminocyclopropan-1-carbonsäurederivat ein Gemisch von Stereoisomeren ist;

c) Trennung des Stereoisomerengemischs durch physikalische Mittel in E- und Z-Diastereomere, worin die E- und Z-Diastereomere ein Enantiomerenpaar umfassen;

d) Trennung des Enantiomerenpaares durch übliche Trennmittel, um ein stereospezifisches 1-Aminocyclopropan-1-carbonsäurederivat herzustellen;

e) Deblockierung des Vorprodukts zur Herstellung einer stereospezifischen 1-Aminocyclopropan-1-carbonsäure mit der Formel

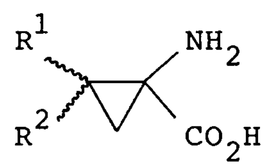

**2.** Ein Verfahren nach Anspruch 1, worin das stereospezifische 1-Aminocyclopropan-1-carbonsäurederivat C-terminal deblockiert wird, um eine stereospezifische Cyclopropylsäure der Formel

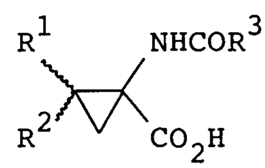

herzustellen.

**3.** Ein Verfahren nach Anspruch 1, worin die N-terminale Deblockierung einer C-terminalen Deblockierung vorausgeht, um die stereospezifische 1-Aminocyclopropan-1-carbonsäure herzustellen.

**4.** Ein Verfahren nach Anspruch 1, worin $R^1$ jeweils $(CH_3)_2CH$ und $R^2$ H ist oder worin jeweils $R^2$ $(CH_3)$-

$_2CH$ und $R^1$ H ist, um eine 2-Isopropyl-1-aminocyclopropan-1-carbonsäure, ein Leucinanalogon, herzustellen.

**5.** Ein Verfahren nach Anspruch 1, worin jeweils $R^1$ $CO_2H$ und $R^2$ H ist oder worin jeweils $R^2$ $CO_2H$ und $R^1$ H ist, die Carboxylgruppe in $R^1$ oder $R^2$ wird, während der Synthese durch übliche Mittel geschützt, um eine 1-Aminocyclopropan-1,2-dicarbonsäure, ein Asparaginanalogon, herzustellen.

**6.** Ein Verfahren nach Anspruch 1, worin jeweils $R^1$ $C_6H_5$ und $R^2$ H ist oder worin jeweils $R^2$ $C_6H_5$ und $R^1$ H ist, um eine 2-phenyl-1-aminocyclopropan-1-carbonsäure, ein Phenylalaninanalogon, herzustellen.

**7.** Ein Verfahren nach Anspruch 1, worin jeweils $R^1$ 4-$HOC_6H_4$ und $R^2$ H ist oder worin jeweils $R^2$ 4-$HOC_6H_4$ und $R^1$ H ist, die Phenolhydroxylgruppe in $R^1$ oder $R^2$ während der Synthese durch übliche Mittel geschützt wird, um eine 2-(4-Hydroxyphenyl)-1-aminocyclopropan-1-carbonsäure, ein Tyrosinanalogon, herzustellen.

**8.** Ein Verfahren nach Anspruch 1, worin jeweils $R^1$ 3-Indolyl und $R^2$ H ist oder worin jeweils $R^2$ 3-Indolyl und $R^1$ H ist, um eine 2-(3-Indolyl)-1-aminocyclopropan-1-carbonsäure, ein Tryptophananalogon, herzustellen.

**9.** Ein Verfahren zur Synthese eines Peptids mit wenigstens zwei Aminosäureresten, ausgewählt aus der aus D- oder L-Isomeren von Aminosäureresten bestehenden Gruppe, worin wenigstens ein Aminosäurerest ein stereospezifischer 1-Aminocyclopropan-1-carbonsäurerest ist, das folgende Schritte umfaßt:

a) Umsetzung einer Diazoverbindung mit der Formel $R^1R^2CN_2$, worin $R^1$ und $R^2$ in beliebiger Reihenfolge ein Substituentenpaar sind, ausgewählt aus der Gruppe, bestehend aus $-CH_3$ und $-CH_3$, $-CH(CH_3)_2$ und -H, $-CO_2H$ und -H, $-CH_2CO_2H$ und -H, $-CH_2SCH_3$ und -H, $-(CH_2)_3NH_2$ und -H, $-CH_2CH_2NHC(=NH)NH_2$ und -H, $-(CH_2)_2NH_2$ und -H, $-C_6H_5$ und -H, -H und -H, 4-Hydroxyphenyl und -H, 3,4-Dihydroxyphenyl und -H, 3-Indolyl und -H, 5-Hydroxy-3-indolyl und -H, -OH und -H, -SH und -H, $-CH_2SH$ und -H, $-CH_2OH$ und -H und 4(5)-Imidazolyl und -H, mit einem Dehydroalaninderivat der Formel

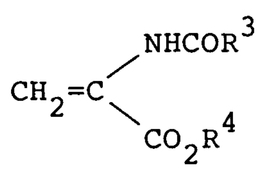

worin $R^3$ aus der aus einer Alkylgruppe, einer aromatischen Gruppe, einer Alkoxygruppe und einer Aryloxygruppe bestehenden Gruppe ausgewählt ist und worin $R^4$ aus der aus einer Alkylgruppe und einer Arylgruppe bestehenden Gruppe ausgewählt ist, um ein Vorprodukt herzustellen;

b) Abbau des Vorprodukts, um ein 1-Aminocyclopropan-1-carbonsäurederivat der Formel

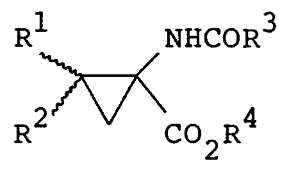

herzustellen, worin das 1-Aminocyclopropan-1-carbonsäurederivat ein Gemisch von Stereoisomeren ist;

c) Trennung des Stereoisomerengemischs durch physikalische Mittel in E- und Z-Diastereomere, worin die E- und und Z-Diastereomere ein Enantiomerenpaar umfassen;

d) Trennung des Enantiomerenpaares durch übliche Trennmittel, um ein stereospezifisches 1-Aminocyclopropan-1-carbonsäurederivat herzustellen;

e) Deblockierung des 1-Aminocyclopropan-1-carbonsäurederivats, um eine N-terminal blockierte stereospezifische 1-Aminocyclopropan-1-carbonsäure herzustellen;

f) Verknüpfung der N-terminal blockierten stereospezifischen 1-Aminocyclopropan-1-carbonsäure mit

einer C-terminal blockierten Aminosäure oder einem Peptid; und
g) Wiederholung der oben genannten Verfahrensschritte, wie sie zur Herstellung des gewünschten Peptids notwendig sind.

**10.** Ein Verfahren nach Anspruch 9 zur Synthese von L-Aspartyl-alpha-aminocyclopropancarbonsäure-propylester.

**11.** Verwendung von Asp-∇Ala•$OC_3H_7$ als Nahrungsmittel und Getränkesüßstoff.